# EUROPEAN PATENT SPECIFICATION

(11) **EP 2 627 691 B1**
(45) Date of publication and mention of the grant of the patent: **25.11.2020**
(21) Application number: 11833429.1
(22) Date of filing: 13.10.2011
(51) Int. Cl.: C08G 64/00

(54) **DEGRADABLE POLYCARBONATES**
ABBAUBARE POLYCARBONATE
POLYCARBONATES DÉGRADABLES

(30) Priority: 13.10.2010 US 392893 P
(43) Date of publication of application: 21.08.2013
(73) Proprietor: Texas A&M University, College Station, TX 77842-3255 (US)
(72) Inventor: STREFF, Jennifer, College Station TX 77840 (US); KRISTUFEK, Samantha, College Station TX 77840 (US); HEARON, Michael, Keith II, Augusta GA 30909 (US); WOOLEY, Karen, College Station TX 77840 (US); LONNECKER, Alexander, College Station TX 77840 (US); BESSET, Celine, F-80 000 Amiens (FR)
(74) Representative: Marks & Clerk LLP Cambridge
(86) International application number: PCT/US2011/056204
(87) International publication number: WO 2012/051448

(56) References cited:
- WO-A1-93/20126
- WO-A1-2010/028362
- US-A1- 2008 026 076
- US-A1- 2009 105 444
- M. GRACIA GARCÍA-MARTÍN ET AL: "Carbohydrate-Based Polycarbonates. Synthesis, Structure, and Biodegradation Studies", MACROMOLECULES, vol. 38, no. 21, 1 October 2005 (2005-10-01), pages 8664-8670, XP055307989, US ISSN: 0024-9297, DOI: 10.1021/ma050676k
- SHENG Y. ET AL.: 'An active ingredient of Cat's Claw water extracts Identification and efficacy of quinic acid' JOURNAL OF ETHNOPHARMACOLOGY vol. 96, 2005, pages 577 - 584, XP027757137

## Description

### Statement of Government Interest

This invention was made with government support under Grant No. CHE-1057441 awarded by the National Science Foundation. The government has certain rights in the invention.

### Cross Reference to Related Applications

This application claims the benefit of U.S. Provisional Application No. 61/392,893, filed October 13, 2010.

### Background

Over the past few decades, there has been a significant interest in the preparation of polymers that originate from renewable resources, typically based upon carbohydrates or fatty acids, in order to diminish the dependence on petroleum products, and also that undergo degradation, to reduce landfill accumulation of waste. Garcia et al., (Macromolecules 2005, 38, 21, 8664-8670) describes the synthesis of linear homo- and co-polycarbonates from sugar-based diols. US 2009/105444 describes a method of making a polycarbonate polymer comprising isosorbide carbonate units. Polycarbonates, especially those based upon bisphenol A, are a foundation material for engineering applications. Many of these polycarbonates, however, form bioresorbable degradation products that are toxic and/or carcinogenic. Degradeable polymers also find use in biomedicine (e.g., sutures, orthopedic devices, tissue engineering, drug delivery devices, etc.), where incorporation of biocompatibility and biodegradability is important. For this purpose, the polymers used are typically of esters, which undergo hydrolysis to afford products containing carboxylic acid and alcohol groups, or carbonates, especially aliphatic carbonates that undergo hydrolytic degradation to give carbon dioxide and alcohols. WO 93/20126 describes a biodegradable and biocompatible polycarbonate comprising (C₃-C₁₀) alkylene carbonic acid units, which may be used as matrices for sustained release of pharmacologically active compounds.

Biomass sources of raw materials provide a diversity of functionalities. For polymers, however, the most extensive versatility arises when the synthetic approach begins from the small molecule monomers, as natural polymers can be difficult to purify (e.g., to avoid immunogenic effects) and they have certain limitations imposed by having the backbone already in place. Small molecule natural products, in contrast, are more readily isolated as pure entities that can then be manipulated in their chemical functionality, polymerized via various types and numbers of linkages to establish the polymer backbones of controlled compositions and architectures, and then undergo further chemical modification and physical processing. An attractive characteristic of both natural and synthetic polymers derived from renewable feedstocks is that they are most often degradable by photochemical, thermal, or hydrolytic means.

### Summary

The present disclosure generally relates to polymer chemistry. More particularly, the present disclosure relates to degradable polycarbonates formed from polyhydroxyl monomers.

There is interest in polymers that originate from renewable resources, in order to, among other things, diminish the dependence on petroleum products and to reduce landfill accumulation of waste. Although nature has several examples of engineering-type construction materials (e.g. cellulose, chitin, etc.) that are degradable, resorbable, and recyclable, most synthetic materials that are designed to be derived from renewable resources and that are degradable are from petrochemicals. The present disclosure provides novel polycarbonate compositions formed from polyhydroxyl monomers which are linked by a carbonate moiety. The present disclosure also provides methods for making polycarbonates.

In one embodiment, the present invention provides a composition comprising a polycarbonate formed from polyhydroxyl monomers which are linked by a carbonate moiety, wherein the polyhydroxyl monomers comprise a polyhydroxyl monomer of formula (1): 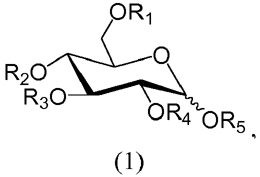
wherein R₁, R₂, R₃, and R₄ are each independently selected from H, a protecting group (e.g., a benzylic group, TBS, and TMS), or COCl;
R₅ is independently selected from H, a protecting group, COCl, or 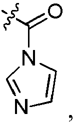 and wherein the polyhydroxyl monomer is not glucose.

Also described herein but not within the scope of the invention are polyhydroxyl monomers according to the following formula: 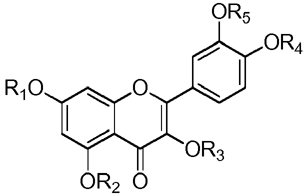 wherein R₁, R₂, R₃, R₄, and R₅ are each independently selected from H, a protecting group, or a reactive carbonyl unit.

Also described herein but not within the scope of the invention are polyhydroxyl monomers according to the following formula: 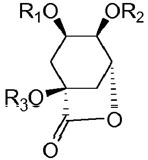 wherein R₁, R₂, and R₃ are each independently selected from H, a protecting group, 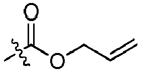 , COCl,

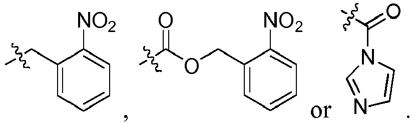

In one embodiment, the present invention provides a method comprising: providing a polyhydroxyl monomer and a carbonylation agent; and polymerizing the polyhydroxyl monomer in the presence of the carbonylation agent so as to form a polycarbonate,
wherein the polyhydroxyl monomer comprises a polyhydroxyl monomer of formula (1): 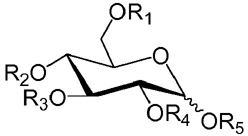 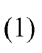 wherein
R₁, R₂, R₃, and R₄ are each independently selected from H, a protecting group (e.g., a benzylic group, TBS, and TMS), or COCl;
R₅ is independently selected from H, a protecting group, COCl, or 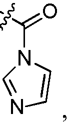 and wherein the polyhydroxyl monomer is not glucose.

The features and advantages of the present invention will be apparent to those skilled in the art.

### Drawings

Some specific example embodiments of the disclosure may be understood by referring, in part, to the following description and the accompanying drawings.
Figure 1 shows an overlay of GPC traces (DMF as eluent, 1 mL/min) for the monomer **1** and its polymerization with diphosgene or phosgene at different concentrations.
Figure 2 shows ¹H NMR (CDCl₃) spectra of diol quinic acid monomers (a) **2** and (b) **3,** and poly(quinic acid carbonate)s (c) **4** and (d) **5**.
Figure 3 shows the effect of each factor evaluated during the experimental design.
Figure 4A shows ¹H NMR spectra for the 1,4 Monomer, (1R,3R,4S,5R)-5-*tert-*butyldimethylsilyloxy-1,4-dihydroxy-cyclohexane-1,3-carbolactone, **2**.
Figure 4B shows ¹³C NMR spectra for the 1,4 Monomer, (1R,3R,4S,5R)-5-*tert-*butyldimethylsilyloxy-1,4-dihydroxy-cyclohexane-1,3-carbolactone, **2.**
Figure 4C shows 2-D (COSY and Hetcor) NMR spectra for the 1,4 Monomer, (1R,3R,4S,5R)-5-*tert*-butyldimethylsilyloxy-1,4-dihydroxy-cyclohexane-1,3-carbolactone, **2.**
Figure 5A shows ¹H NMR spectra for the Poly(1,4-quinic acid carbonate), **4**.
Figure 5B shows 2-D (COSY) NMR spectra for the Poly(1,4-quinic acid carbonate), **4.**
Figure 6A shows ¹H NMR spectra for the 1,5 Monomer, (1R,3R,4S,5R)-4-*tert-*butyldimethylsilyloxy-1,4-dihydroxy-cyclohexane-1,3-carbolactone, **3.**
Figure 6B shows ¹³C NMR spectra for the 1,5 Monomer, (1R,3R,4S,5R)-4-*tert-*butyldimethylsilyloxy-1,4-dihydroxy-cyclohexane-1,3-carbolactone, **3.**
Figure 6C shows 2-D (COSY and Hetcor) NMR spectra for the 1,5 Monomer, (1R,3R,4S,5R)-4-*tert*-butyldimethylsilyloxy-1,4-dihydroxy-cyclohexane-1,3-carbolactone, **3**.
Figure 7A shows ¹H NMR spectra for the Poly(1,5-quinic acid carbonate), **5.**
Figure 7B shows 2-D (COSY) NMR spectra for the Poly(1,5-quinic acid carbonate), **5.**

While the present disclosure is susceptible to various modifications and alternative forms, specific example embodiments have been shown in the figures and are herein described in more detail. It should be understood, however, that the description of specific example embodiments is not intended to limit the invention to the particular forms disclosed, but on the contrary, this disclosure is to cover all modifications as illustrated, in part, by the appended claims.

### Description

In certain embodiments, the present disclosure provides hydrolytically-degradable polycarbonates. Such polycarbonates are hydrolytically labile and may release a polyhydroxyl monomer and carbon dioxide upon degradation. The polycarbonates of the present disclosure may benefit from useful physical properties, such as improved and/or useful mechanical and/or thermal properties.

Also described herein are uses of the polycarbonates of the present disclosure in any application in which polycarbonate or a polycarbonate-like material may be useful. For example, the polycarbonates of the present disclosure may be used in applications such as orthopedic devices, sutures, biomedical implants, indwelling medical devices, drug delivery systems and/or matricies, replacement of polyesters and non-hydrolysable polycarbonates. The polycarbonates of the present disclosure may be used in any type of plastic that is to undergo degradation to be environmentally-resorbable, as well as in engineering materials (e.g. for replacement of bisphenol A polycarbonate materials) even without the need for rapid or accelerated degradation.

In general, the polycarbonates of the present disclosure comprise two or more polyhydroxyl monomer repeating units that may be linked by a carbonate moiety. The polycarbonates also may include networks of more than one polycarbonate molecule (e.g., crosslinked polycarbonate networks), as well as crosslinks between polycarbonate molecules.

The polycarbonates of the present disclosure may be represented by the formula: 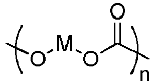 wherein O-M-O represents a polyhydroxyl monomer repeating unit and n is >1. Hydrolysis of the polycarbonates of the present disclosure may be represented by the formula:

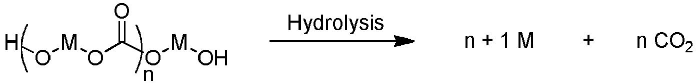

As used herein, the term "polyhydroxyl monomer" refers to any molecule having two or more hydroxyl groups and may be monomeric or polymeric. In general, suitable polyhydroxyl monomers are those polyhydroxyl monomers that are capable of reacting with a carbonylation agent to form a polycarbonate. In certain embodiments, a suitable polyhydroxyl monomer may be a natural product (e.g., derived and/or sourced from natural feedstocks). By using polyhydroxyl monomers that are natural products, the resulting polycarbonates will, among other things, be less dependent on petrochemicals, have tunable mechanical properties for multiple types of applications, and undergo degradation (e.g., hydrolysis) to release those natural products. Examples of suitable polyhydroxyl monomers that are natural products, include, but are not limited to, saccharides such as, for example, glucose (e.g., D-glucose), mannose, fructose, sucrose, lactose, raffinose, stachylose, and the like; polyphenolic molecules such as, for example, quercetin, catechin, epicatechin, epicatechin 3-gallate, epgallocatechin, epigallocatechin 3-gallate, tannin, teaflavins, and other such flavonoids, and other polyphenolic natural products that derive from teas and other sources; and derivatives of polyphenolic molecules such as, for example quinic acid (derived from chlorogenic acid). The polycarbonates of the present disclosure may include more than one type of polyhydroxyl monomer.

The polycarbonates of the present disclosure may be formed using one or more of cabonylation, protection, ring opening polymerization (ROP), copolymerization, and deprotection. For example, in certain embodiments, the polycarbonates of the present disclosure may be formed via AA + BB copolymerizations of the respective protected polyhydroxyl monomer with a carbonylation agent; and, ter- and higher copolymerizations may be used. In other embodiments, ring opening copolymerizations may be used, as well as AB copolymerizations, di-, ter- and higher copolymerizations.

Carbonylation may occur by reacting a polyhydroxyl monomer of the present disclosure with a carbonylation agent. Examples of suitable carbonylation agents include, but are not limited to, phosgene, diphosgene, and triphosgene, and derivaties of phosgene (e.g., such as those shown in the examples below), carbonyldiimidazole, bis(4-nitrophenyl)carbonate, and other common reagents. For cyclic ring opening polymerization of epoxide monomers, carbon dioxide may be used as the carbonylation agent.

Given that a polyhydroxyl monomer may have multiple reactive hydroxyl groups, the methods used to form the polycarbonates of the present disclosure may employ protecting groups to influence the number and placement of the reactive hydroxyl groups through which polymerization may occur. Accordingly, in certain embodiments, polyhydroxyl monomers may include one or more protecting groups. Examples of suitable protecting groups include, but are not limited to, silyl ethers (e.g., trimethylsilyl (TMS), *tert*-butyldiphenylsilyl (TBDPS), *tert-*butyldimethylsilyl (TBS/TBDMS) triisopropylsilyl (TIPS), and [2-(trimethylsilyl)ethoxy]methyl (SEM)), benzyl ethers, methoxymethyl ethers, and other ether, ester or protecting groups for hydroxyl (alcohol and phenol) groups, and photolabile protecting groups suitable for hydroxyl moieties (e.g., o-nitrobenzyl ethers, and m-nitrophenyl carbamate).

In some embodiments, protecting groups may be located in various combinations of hydroxyl groups to form regioisomeric monomers. In this way, one or more regiosiomeric polycarbonates may be formed. In addition, by protecting certain hydroxyl groups while allowing other hydroxyl groups to remain reactive, the architecture of the resulting polycarbonate can be varied from linear to branched, hyperbranched, or crosslinked.

Deprotection may be effected by any suitable technique. For example, a silyl ether protecting group may be removed using trifluoroacetic acid (TFA), tetra-*n*-butylammonium fluoride (TBAF), boron trifluoride etherate (BF₃-OEt₂), and other commonly employed conditions. For removal of a photolabile protecting group, light, such as UV light, may be used. One of ordinary skill in the art will be able to determine a suitable means for deprotection based, at least in part, on the protecting group to be removed.

The polycarbonates of the present disclosure may be tailored to have a broad range of physical and mechanical properties. For example, differences in regiochemistry (e.g., in the case of D-glucose, 1,4- vs. 1,6- vs. 1,2-), stereochemistry (e.g., in the case of D-glucose, α vs. β vs. mixtures of α and β), and architecture (linear vs. hyperbranching with AB₂, AB₃, and AB₄). There is additional ability to alter the chain end functionalities through chemical modification reactions performed on the polycarbonates of the present disclosure. Additionally, different degradation kinetics may be achieved. Furthermore, the polycarbonates of the present disclosure may be tailored by forming polymer networks, including by crosslinking polycarbonate chains. In some embodiments, the polycarbonates of the present disclosure have a molecular weight of from 1 kDa to 500 kDa, 5 kDa to 300 kDa, or 10 kDa to 200 kDa.

As mentioned above, the polycarbonates of the present disclosure may be used to form a polycarbonate network. In general, such polycarbonate networks comprise two or more polycarbonates associated into a polymer network. In some embodiments, the polycarbonate network may be an interpenetrating polymer network or semi-interpenetrating polymer network. In certain embodiments, a crosslinked polycarbonate network may be formed by providing one or more linear polycarbonates followed by forming one or more crosslinks between another linear polycarbonate. A crosslinked polycarbonate network may also be formed by direct crosslinking of multifunctional comonomers. The crosslinks may be formed using any technique. For example, the crosslinks may be formed using chemical routes. The crosslinks also may be formed using photo-initiated reactions (e.g., thiol-ene reactions) on functionalized polymers and/or monomers.

In certain embodiments, the present disclosure provides a polyhydroxyl monomer according to formula 1: 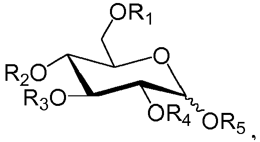 wherein R₁, R₂, R₃, and R₄ are each independently selected from H, a protecting group (e.g., a benzylic group, TBS, and TMS), or COCl; R₅ is independently selected from H, a protecting group, COCl, or 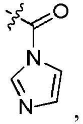 and wherein the polyhydroxyl monomer is not glucose. Examples of such polyhydroxyl monomers include, but are not limited to: 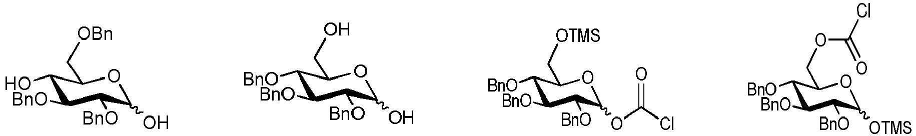 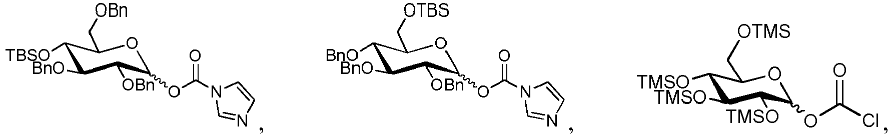 and

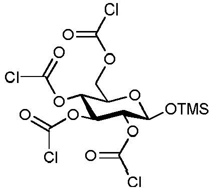

Also described herein is a polyhydroxyl monomer according to formula 2: 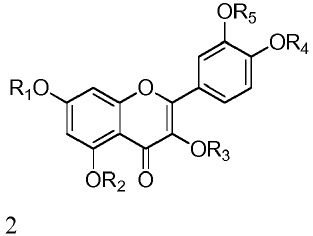 wherein R₁, R₂, R₃, R₄, and R₅ are each independently selected from H, a protecting group, or a reactive carbonyl unit (e.g. COCl or 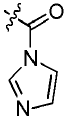 ). Examples of such polyhydroxyl monomers include, but are not limited to:

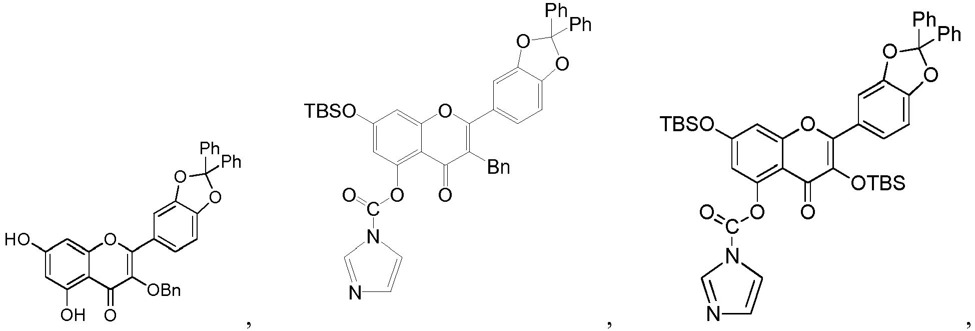

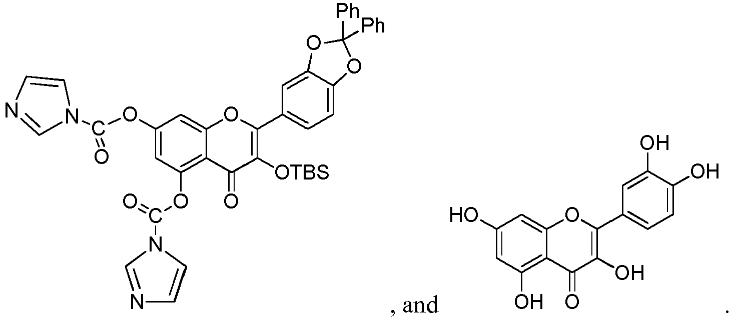

Also described herein is a polyhydroxyl monomer according to formula 3: 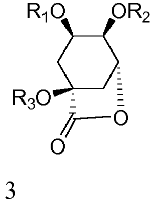 wherein R₁, R₂, and R₃ are each independently selected from H, a protecting group, 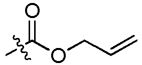 , COCl, 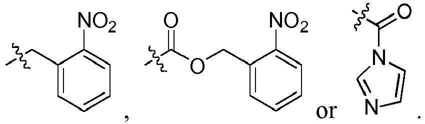 Examples of such polyhydroxyl monomers include, but are not limited to:

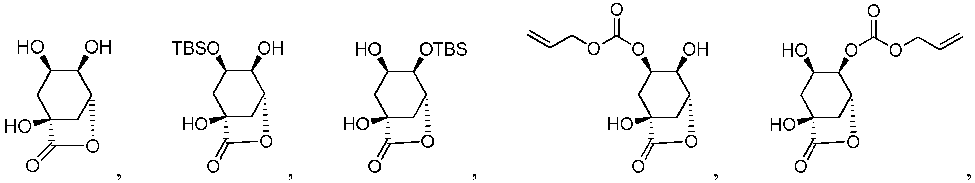

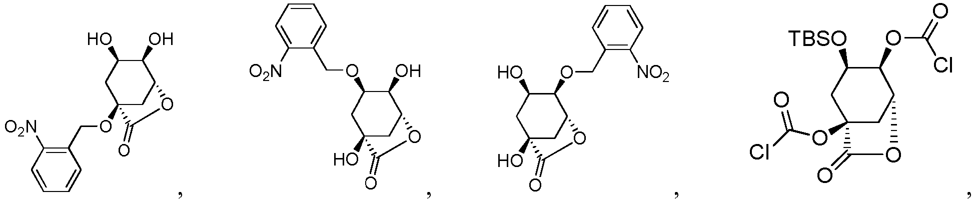

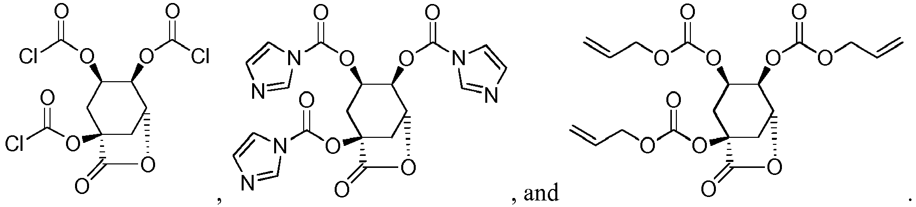

To facilitate a better understanding of the present invention, the following examples of certain aspects of some embodiments are given. In no way should the following examples be read to limit, or define, the entire scope of the invention.

### Examples

### Example-Degradable polycarbonates

A series of diol monomers, starting from glucose, 1, and quinic acid, 2, were initially prepared using selective protecting group installations and removals combined with functional group transformations. Polycarbonates were synthesized, using these monomers by condensation with carbonylation agents. A general strategy for the synthesis of polycarbonates by condensation with carbonylation agents of 1,4-diol and 1,6-diol monomers of D-glucose, respectively, 3 and 4 and the 1,4-diol monomer of 1,3-lactone quinic acid, 5 is shown below.

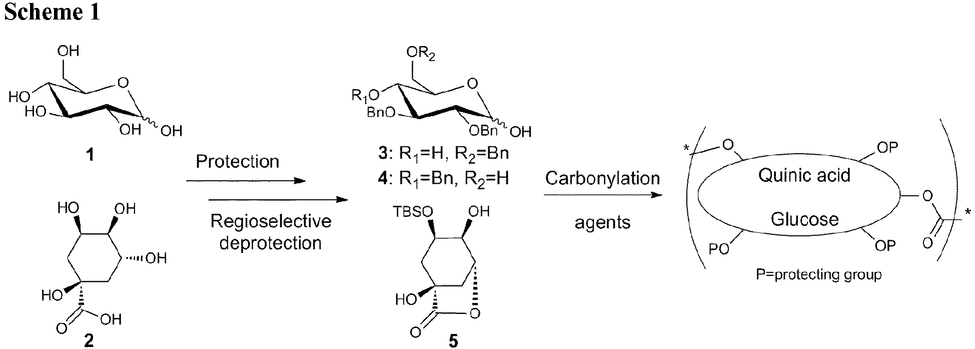

Other polymerizations, especially ring opening polymerization, can alternatively be applied to afford biodegradable saccharide- and quinic acid-based polymers (for example, as shown below in **Scheme 2** for the synthesis of polycarbonates by ring opening polymerizations of 1,2-anhydroglucose monomer, **6** and 4,5-carbonate quinic acid, 7). For example, ring opening copolymerization with carbon dioxide using Darensbourg's chromium catalyst may be applied to the protected 1,2-anhydroglucose monomer, **6.** Ring opening polymerization, in the presence of diazabicyclo[5.4.0]undec-7-ene, as an efficient catalyst of a five- membered cyclic carbonate, was tested on the protected 1,3-lactone-4,5-carbonate quinic acid, 7.

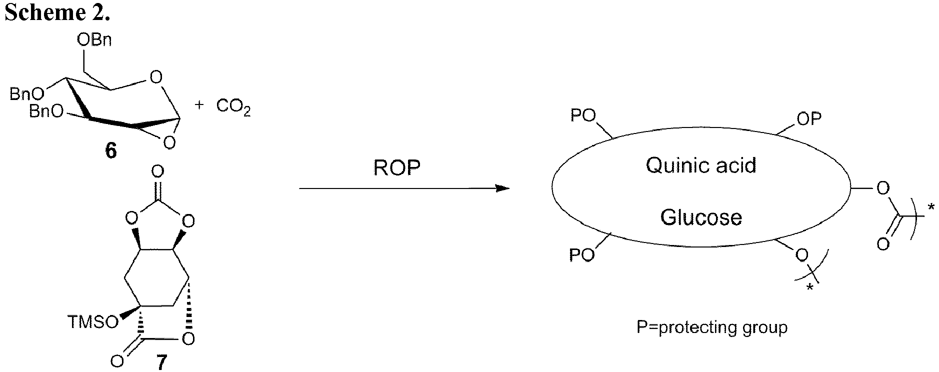

The conditions for polymerization of each monomer have been investigated and molecular weights between 7 and 27 kDa have been reached for poly(quinic acid carbonate)s. Thermal properties have also been evaluated for these polymers, showing high decomposition temperature (onset above 350 °C) and glass transition temperature (T_{g} = 205 °C), confirming the excellent stability and rigidity of the bicyclic backbone present in the monomer unit, **5** and **7.**

Unique, potentially biodegradable and bioresorbable, polycarbonates, starting from renewable resources, glucose or quinic acid, have been synthesized by condensation or ring opening polymerizations.

### Example-Linear Poly(D-glucose carbonate)s via AA + BB Copolymerizations

The α and β isomers of 1,4-diol monomers of D-glucose having positions 2, 3, and 6 protected with benzylic groups, **5** or **8,** were prepared according to **Scheme 3**. Installation of a benzylidene at positions 4 and 6 of a mixture of α and β isomers of glucose having an allyl protecting group at the anomeric site (position 1), **1,** gave the α isomer in 41% yield and the β isomer in 34% yield, after separation. Each isomer was then carried forward with reactions involving benzylation of the 3 and 4 positions, and opening of the benzylidene by reaction with sodium cyanoborohydride and aluminum trichloride in tetrahydrofuran in the presence of 3 Å molecular sieves to give **4a** and **7.** Removal of the allyl protecting groups then afforded the α and β isomers of 1,4-diol monomers of D-glucose. Similarly, the 1,6-diol monomer of D-glucose having positions 2, 3, and 4 protected with benzylic groups was also prepared from **4b.** Protection of the anomeric position alternatively may use 1-O-methyl protecting group in place of the allyl.

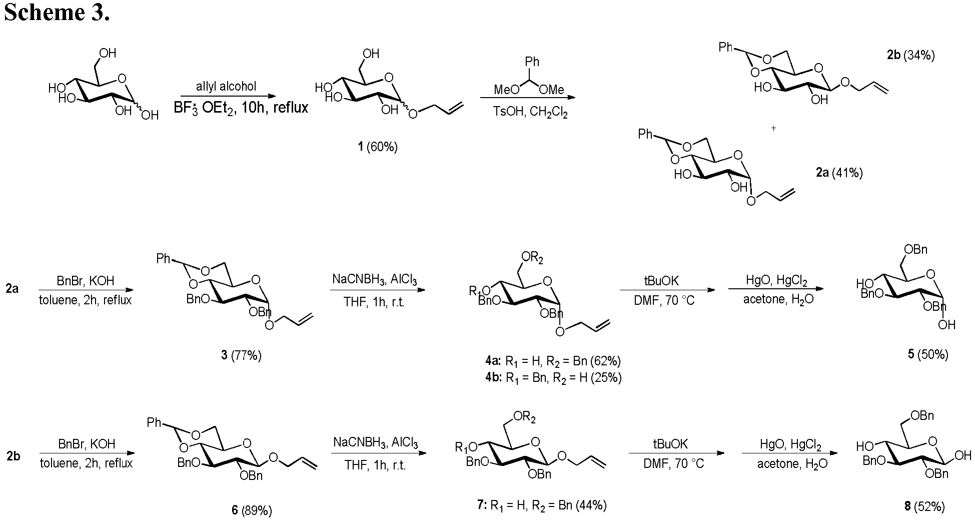

### Synthesis of allyl D-glucopyranoside (1)

D-(+) Glucose (1.072 g, 5.935 mmol) and boron trifluoride diethyl etherate (0.253 g, 1.78 mmol) were added to 30 mL of allyl alcohol and heated to reflux. The reaction was mantained for 10 hr resulting in a dark amber color. The reaction was allowed to cool to r.t. and then neutralized with triethyl amine. Solvents were evaporated under high vacuum and the residue was purified by column chromatography on silica gel, eluting with a 9:1 mixture of dichloromethane and methanol, to yield a colorless oil (0.7854 g, 60%).1H NMR shows the oil to be a 73:27 mixture of α- and β-allyl D-glucopyranoside.

### Synthesis of allyl 4,6-benzylidene-α-D-glucopyranoside (2a) and 4,6-benzylidene-p-D-glucopyranoside (2b)

Allyl D-glucopyranoside (0.5 g, 2 mmol), toluenesulfonic acid (0.01955 g, 0.1135 mmol), benzylidene dimethyl acetal (0.852 mL g, 5.68 mmol) and 10 mL dry dichloromethane were mixed in a 100 mL round bottomed flask and charged with nitrogen. The solution was allowed to mix at r.t. until all components were fully dissolved. The reaction was allowed to proceed at r.t. for 16 hr, 30 °C for 6 hours and then r.t. for 12 hr. An additional equivalent of benzylidene dimethyl acetal (0.35 mL, 2.3 mmol) was added after 22 hr. The reaction was purified by column chromatography on silica gel, eluting with a 8:2 mixture of ethyl acetate and hexanes to produce allyl 4,6-benzylidene-α-D-glucopyranoside(0.2061 g, 41%) and allyl 4,6-benzylidene-β-D-glucopyranoside (0.1715 g, 34%) as white solids.

Allyl 4,6-benzylidene-α-D-glucopyranoside (2a): ¹H NMR (CDCl₃) δ: 7.35-7.51 (m, 5H, Ar), 5.87-6.00 (m, 1H, allyl), 5.54 (s, 1H, benzylidene), 5.24-5.36 (dddd, 2H, allyl), 4.96-4.97 (d, 1H, J=4.0 Hz, H-2), 4.23-4.32 (m, 2H, O-CH₂-allyl), 4.04-4.10 (dd, 1H, CH₂), 3.94-4.00 (t, 1H, CH), 3.85-3.90 (dd, 1H, CH₂), 3.71-3.82 (ddd, 1H, CH), 3.61-3.69 (dd, 1H, CH), 3.47-3.54 (t, 1H, CH), 2.69-2.69 (d, 1H, J = 2.0 Hz, OH), 2.20-2.24 (d, 1H, J =10.0 Hz, OH)

Allyl 4,6-benzylidene-β-D-glucopyranoside (2b): ¹H NMR (CDCl₃) δ: 7.34-7.49 (m, 5H, Ar), 5.86-5.99 (m, 1H, allyl), 5.52 (s, 1H, benzylidene), 5.21-5.36 (dddd, 2H, allyl), 4.44-4.47 (d, 1H, J = 7.7 Hz, H-2), 4.39-4.41 (d, 1H, CH1), 4.10-4.37 (dddd, 2H, O-CH₂-allyl), 3.81-3.86 (dd, 1H, CH₂), 3.75-3.79, (dd, 1H, CH₂), 3.53-3.59 (t, 1H, CH), 3.49-3.50 (ddd, 1H, CH), 3.44-3.48 (t, 1H, CH), 2.66-2.68 (d, 1H, OH), 2.50-2.52 (d, 1H, OH).

### Synthesis of allyl 2,3-di-O-benzyl-4,6-benzylidene-β-D-glucopyranoside (3)

Allyl 4,6-benzylidene-α-D-glucopyranoside (0.1356 g, 0.4369 mmol), potassium hydroxide (0.1679 g, 2.991 mmol), and benzyl bromide (0.0533 g, 3.12 mmol) were suspended in 10 mL of toluene and heated to reflux for two hours. The reaction mixture was allowed to cool to room temperature, washed with water (50 mL), and extracted with dichloromethane (50 mL). The organic layers were combined, dried with magnesium sulfate and concentrated under vacuum. Crude product was purified by column chromatography over silica gel to afford allyl 2,3-di-O-benzyl-4,6-benzylidene-α-D-glucopyranoside as a white solid (77%).

### Synthesis of allyl 2,3,6-tri-O-benzyl-α-D-glucopyranoside(4a) and allyl 2,3,4-tri-O-benzyl-α-D-glucopyranoside(4b)

Allyl 2,3-di-*O*-benzyl-4,6-benzylidene-α-D-glucopyranoside (0.0954 g, 0.195 mmol) was dissolved in dry tetrahydrofuran in a flame dried flask and charged with nitrogen. Sodium cyanoborohydride (0.0973 g, 1.55 mmol) was added under nitrogen and allowed to stir for 45 min. Aluminum trichloride (0.2067 g, 1.550 mmol) was added under nitrogen at 0°C and allowed to stir for one hour. TLC indicated that the reaction was not complete and heated to 30°C for 30 minutes, 40°C for 30 minutes, 45°C for 30 minutes, 55°C for 225 minutes, and then allowed to stir at room temperature overnight. The solution was filtered to remove salts and diluted with dichloromethane (10 ml). The mixture was washed with water (10 mL). The aqueous layer was extracted with 10 mL(x2) of dichloromethane. The organic layers were dried with magnesium sulfate and concentrated under vacuum. The oily residue was purified by column chromatography over silica gel (7:3 hexanes/ethyl acetate) to yield allyl 2,3,6-tri-*O*-benzyl-α-D-glucopyranoside (62%) and allyl 2,3,4-tri-*O*-benzyl-α-D-glucopyranoside (25%) as clear oils.

### Synthesis of 2,3,6-tri-O-benzyl-α-D-glucopyranoside (5)

Allyl 2,3,6-tri-*O*-benzyl-α-D-glucopyranoside (0.0954 g, 0.195 mmol) was added to a flame dried flask and charged with nitrogen and dimethyl sulfoxide (2 mL) was added. Potassium *tert-*butoxide (0.0640 g, 0.570 mmol) was added under nitrogen and the solution turned yellow instantly. The reaction mixture was heated to 100 °C and allowed to stir for 2.5 hours. The solution turned black over a few minutes. After cooling to room temperature, the reaction mixture was quenched with 10 mL of water. The solution was extracted with dichloromethane (3x15 mL), the organic layers were combined and washed with water (2x10 mL) and saturated sodium chloride solution (1X10 mL). The organic layer was dried with magnesium sulfate and concentrated under vacuum.

The crude oil was transferred to a 25 mL round bottomed flask and dissolved in acetone (2 mL) and water (0.2 mL). Mercuric oxide (0.0141 g, 0.111 mmol) was added. A solution of mercuric chloride (0.0193 g, 0.071 mmol) in acetone (1 mL) and water (0.1 mL) was added and allowed to stir for one hour. The mixture was filtered to remove mercuric salts and concentrated to 5 mL under vacuum. The solution was extracted with dichloromethane (10 mL) and washed with water (2x10 mL) and saturated sodium chloride solution (2x10 mL). The organic layer was dried with magnesium sulfate and concentrated under vacuum to yield 0.0475 g of white solid (50%).

### Synthesis of allyl 2,3-di-O-benzyl-4,6-benzylidene-α-D-glucopyranoside (6)

Allyl 4,6-benzylidene-β-D-glucopyranoside (0.0597 g, 0.192 mmol), potassium hydroxide (.0739 g, 1.32 mmol), and benzyl bromide (0.02346, 1.372 mmol) were suspended in toluene (6 mL) and heated to reflux for two hours. The reaction was allowed to cool to room temperature and was washed with 20 mL of water and extracted with 20 mL of dichloromethane. The organic layer was dried with magnesium sulfate and concentrated under vacuum. The crude product was purified by column chromatography over silica gel (85:15 hexanes, acetone) to give allyl 2,3-di-*O*-benzyl-4,6-benzylidene-β-D-glucopyranoside as a white solid (88.5%).

### Synthesis of allyl 2,3,6-tri-O-benzyl-β-D-glucopyranoside (7)

Allyl 2,3-di-*O*-benzyl-4,6-benzylidene-β-D-glucopyranoside (0.0612 g, 0.125 mmol) was dissolved in dry tetrahydrofuran and charged with nitrogen. Sodium cyanoborohydride (0.901 g, 0.0566 mmol) was added and allowed to stir for 45 min. Aluminum trichloride (1.101 g, 0.1468 mmol) was added under nitrogen at 0°C and allowed to stir for one hour. The solution was filtered to remove salts and diluted with dichloromethane (10 mL). The mixture was washed with water (10 mL) and the queous layer was extracted with 10 mL of dichloromethane. The organic layers were dried with magnesium sulfate and concentrated under vacuum. The oily residue was purified by column chromatography over silica gel (8:2 hexanes/ethyl acetate) to yield allyl 2,3,6-tri-*O-*benzyl-β-D-glucopyranoside as a clear oil (44.4%).

### Synthesis of 2,3,6-tri-O-benzyl-p-D-glucopyranoside (8)

Allyl 2,3,6-tri-*O*-benzyl-β-D-glucopyranoside (0.02 g, 0.06 mmol) was added to a oven dried flask and charged with nitrogen and dimethyl sulfoxide (1 mL) was added. Potassium *tert-*butoxide (0.0387g, 0.345 mmol) was added under nitrogen and the solution turned yellow instantly. The reaction mixture was heated to 100°C and allowed to stir for 2.5 h. The solution turned black over a few minutes. After cooling to room temperature, the reaction mixture was quenched with 10 mL of water. The solution was extracted with dichloromethane (3x15 mL), the organic layers were combined and washed with water (2x10 mL) and saturated sodium chloride solution (1x10 mL). The organic layer was dried with magnesium sulfate and concentrated under vacuum.

The crude oil was transferred to a 25 mL round bottomed flask and dissolved in acetone (4 mL) and water (0.4 mL). Mercuric oxide (0.0439 g, 0.203 mmol) was added. A solution of mercuric chloride (0.0363 g, 0.134 mmol) in acetone (2 mL) and water (0.2 mL) was added and allowed to stir for one hour. The mixture was filtered to remove mercuric salts and concentrated to 5 mL under vacuum. The solution was extracted with dichloromethane (10 mL) and washed with water (2x10 mL) and saturated sodium chloride solution (2x10 mL). The organic layer was dried with magnesium sulfate and concentrated under vacuum to yield 0.0103 g of white solid (52%).

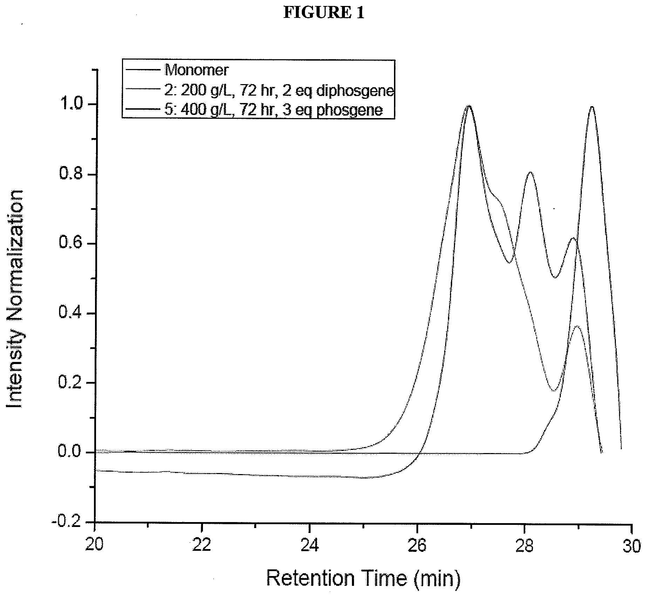

Polymerization of **1** was accomplished by reaction with phosgene, diphosgene, and triphosgene, in the presence of pyridine (**Scheme 4**). As illustrated in **Figure 1****,** the polymerization can be monitored by gel permeation chromatography (GPC).

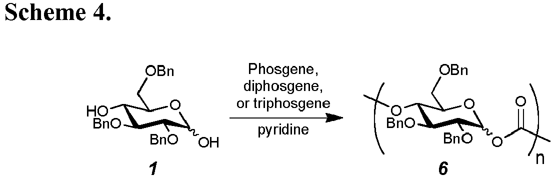

Removal of the benzyl protecting groups of 6 by hydrogenolysis by reaction with H₂ and 10% Pd/C may be used to achive the desired poly(1,4-D-glucose carbonate), **7** (**Scheme 5**).

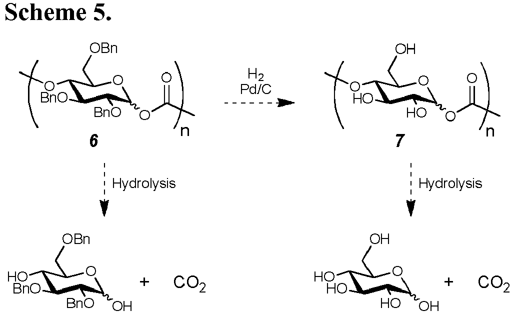

The kinetics of hydrolytic degradation of both **6** and **7** may be determined by GPC, NMR and IR spectroscopy (**Scheme 5**). For GPC analyses, instruments operating with chloroform, tetrahydrofuran, N,N-dimethylformamide and water are available. However, it may be necessary to perform silylation upon the reaction mixtures, as is typically done for sugars, prior to gas chromatographic analysis, by reaction with N-trimethylsilyltrifluoroacetamide, to avoid adsorption of the hydroxyl-containing polymers and degradation products to the column packing material. The deprotected polymer 7 is of key interest. The hydrophobicity/hydrophilicity can be characterized by contact angle measurements. Thermal analysis and scattering studies can be used to probe the crystallinity.

The synthesis, characterization and polymerization of the poly(1,6-D-glucose) isomer (**Scheme 6**) followed the procedures outlined for the 1,4-analog. Allyl 2,3,4-tri-O-benzyl-D-glucopyranoside (**1** of Scheme 6, an α and β isomeric mixture of **4b** of Scheme 3)- was converted to propenyl 2,3,4-tri-O-benzyl-D-glucopyranoside (**2**) by reaction with potassium *tert*-butoxide, followed by removal of the protecting group by reaction with hydrochloric acid (0.2 N aqueous solution) in acetone heated at reflux to afford the 1,6-diol monomer, 2,3,4-tri-O-benzyl-D-glucopyranoside (**3**). Polymerization of **3** with phosgene produced the benzyl-protected polycarbonate **4.** Hydrogenolysis of **4** leads to the polyhydroxyl polycarbonate material, **10** (not shown), that upon hydrolytic degradation will reproduce glucose plus carbon dioxide.

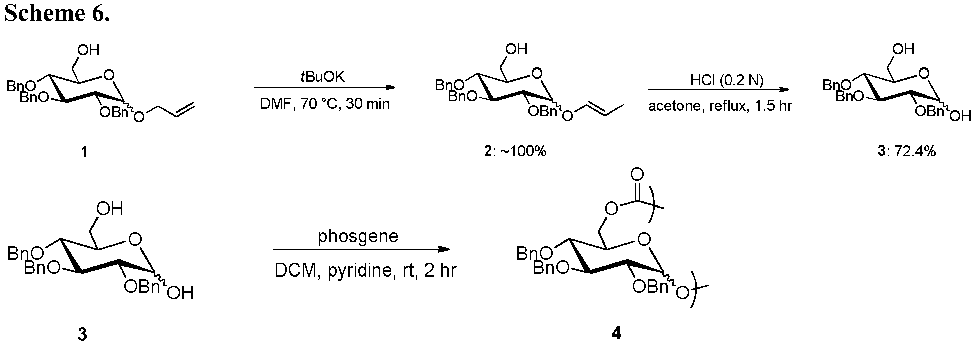

### Synthesis of propenyl 2,3,4-tri-O-benzyl-D-glucopyranoside (2)

Allyl 2,3,4-tri-*O*-benzyl-D-glucopyranoside (3.1 g, 6.3 mmol), **1,** was concentrated under reduced pressure in a flame dried round-bottomed flask and charged with N₂. Dry DMF (11 mL) was syringed into the flask and the contents were heated to 70 °C. When the reaction temperature was reached, potassium *tert*-butoxide (1.4165 g, 12.623 mmol) was added, which immediately turned the solution dark black. After stirring for 30 min, the reaction vessel was cooled in an ice bath and the contents were neutralized with a 4 N HCl aqueous solution. The mixture was extracted with 250 mL of dichloromethane, which was then subsequently washed with water (2 x 250 mL). The organic layer was dried with MgSO₄ and concentrated under reduced pressure to yield the isomer quantitatively (3.1 g). ¹H NMR: (CDCl₃, 300 MHz): δ 7.36-7.28 (m, 15H, Ph-H), 5.99-5.95 (m, 1H, CH=O), 5.29 (s, 0.38H, H-1ax), 5.03-4.99 (d, 1H, CH=C), 4.91-4.56 (m, 6.7H, CH₂-Ph and H-1eq), 4.08 (t, 1H, H-3), 3.77-3.52 (m, 5H, H-2, H-4, H-5, H-6, H-6'), 1.66-1.63 (dd, 3H, CH₃-C).

### Synthesis of 2,3,4-tri-O-benzyl-D-glucopyranoside (3)

Propenyl 2,3,4-tri-*O*-benzyl-D-glucopyranoside (3.1 g, 6.3 mmol), **2,** was dissolved in 30 mL of acetone along with 8 mL of 0.2 N aqueous HCl solution. The mixture was heated to reflux for 1.5 hr. After this time, the condenser was removed from the reaction vessel to concentrate the sample. The resulting solution was extracted with 300 mL of ethyl actetate, which was then washed with 500 mL of water. The organic layer was dried with MgSO₄, and concentrated under reduced pressure to give an amber oil. The crude product was purified by column chromatography over silica gel (5:5 Hex/EtOAc) to give a waxy white solid (2.2461 g, 72.4%). ¹H NMR: (CDCl₃, 300 MHz): δ 7.36-7.27 (m, 15H, Ph-H), 5.19-5.18 (d, 0.55H, H-1ax), 4.97-4.63 (m, 6.45H, CH₂-Ph, H-1eq), 4.01-3.34 (m, 5H, H-3, H-6, H-4, H-2). ¹³C NMR: (CDCl₃, 300 MHz): δ 128.57-126.67 (Ph), 97.35, 91.21, 84.44, 83.21, 81.56, 80.13, 77.34, 75.68, 75.37, 75.03, 74.82, 73.31, 71.01, 61.89.

### Synthesis of 1,6 glucose polycarbonate (4) by polymerization of the 1,6-diol monomer of glucose (3)

In a flame dried 5 mL round bottom flask, 2,3,4-tri-O-benzyl-D-glucopyranoside (0.0785 g, 0.174 mmol), 3, was massed and subjected to reduced pressure to remove residual water. Under N₂, 0.175 mL of dichloromethane was added, followed by pyridine (0.025 mL, 0.33 mmol). After stirring for 30 min, the solution was cooled to 0 °C and 0.058 mL of phosgene solution (60% in toluene) was added dropwise over a 5 min span. The addition of phosgene was completed two more times, adding a total of 0.175 mL of solution (0.333 mmol).

Fractions were quenched after two hours and 24 hours of reaction. To quench the reaction, saturated sodium bicarbonate solution was added to the reaction mixture to neutralize any unreacted phosgene. The reaction mixture was then extracted with dichloromethane. The organic layer was collected and washed with 1 N aqueous HCl solution to remove any excess pyridine. The organic layer was dried with MgSO₄ and concentrated under reduced pressure. Polymer **4** was analyzed by ¹H NMR spectroscopy and size exclusion chromatography. ¹H NMR: (CDCl₃, 300 MHz): δ 7.36-7.27 (m, Ph-H), 5.53-5.41 (m, H-1ax_{poly}), 5.20-5.19 (d, H-1axₘₒₙ), 5.16-5.13 (m, H-1eq_{poly}), 4.99-3.31 (m, CH₂-Ph, H-1eqₘₒₙ, H-2, H-3, H-4, H-5, H-6). SEC: Mₙ = 6970 Da, M_{w} = 7210 Da, PDI = 1.03.

### Example-Linear Poly(saccharide carbonate)s via Ring Opening Copolymerizations

The linear poly(D-glucose carbonate) having a 1,2-linkage is also of significant interest, for its physical and mechanical properties, and characteristics of hydrolytic degradation to glucose and carbon dioxide (**Scheme 7**). The linear poly(1,2-D-glucose carbonate), **11,** may be synthesized by copolymerization of the benzyl protected D-glucose oxirane, **12,** with carbon dioxide using Darensbourg's chromium catalyst, **13,** followed by hydrogenolysis of the intermediate benzyl-protected polymer, **14**. Transformation of the tri-*O*-acetyl glucal, **15**, to the tri-*O*-benzyl glucal, **16,** was accomplished in good yield. The *O*-benzylated glucal is more reactive than is **15** toward epoxidation, performed by the *in situ* generation of dimethyldioxirane (DMDO) from oxone®/acetone in a biphasic system of aqueous NaHCO₃ and CH₂Cl₂. This reaction sequence has led to an excellent yield of the protected 1,2-anhydrosugar monomer, **12.** It is expected that **11** will exhibit properties that are intermediate between those of **7** and **10.**

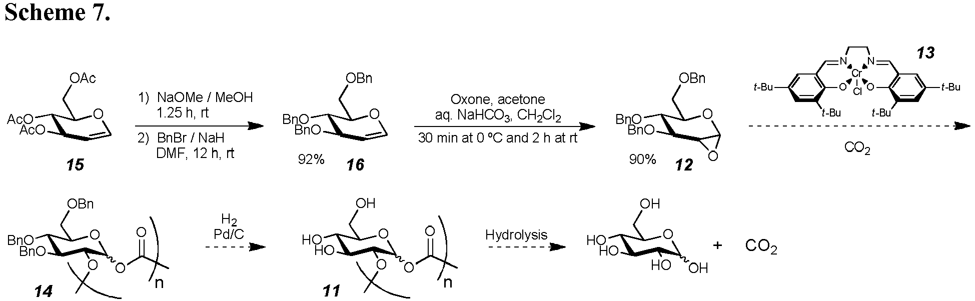

### Example-Linear Poly(saccharide carbonate)s via AB Copolymerizations

The pre-establishment of an AB monomer structure has significant advantage in being able to then perform polymerization directly with a built-in stoichiometric equivalence of the functionalities, often leading to higher degrees of polymerization than obtained by AA + BB polymerizations. As illustrated in **Scheme 8,** the 1,6-diol monomer 8 can be converted into two different AB monomers, **17a** and **17b,** by introduction of a trimethylsilyl protecting group and then installation of the chloroformate. The trimethylsilyl group blocks one of the two hydroxyl groups; a mixture of products **18a** and **18b** are expected. Reaction of **18a** or **18b** with diphosgene in the presence of activated carbon and N,N-dimethylaniline is expected to afford the respective chloroformates without carbonate formation. The trimethylsilyl ether and trimethylsilyl hemiacetal groups of **17a** and **17b,** respectively, also serve as the assisting groups for polycarbonate formation, by releasing the nucleophilic alkoxides, upon reaction with silver fluoride, for attack upon the chloroformates and subsequent elimination of chloride from the tetrahedral intermediate. The chloride ion is removed from the equilibria by precipitation as silver chloride. This chemistry has been applied to the formation of phenyl-based polycarbonates, which are challenging due to the potential for elimination of phenolate from the tetrahedral intermediates. Because of the potential addition/elimination and exchange reactions that are possible, with opening and reclosing of the sugar ring, the stereochemistries of the monomers may not be retained in the polymers.

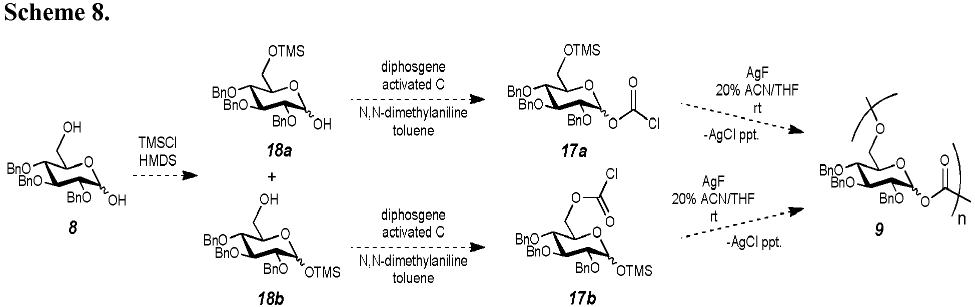

An alternative AB polymerization strategy for both 1,4- and 1,6-glucose-based monomers is illustrated in **Scheme 9.**

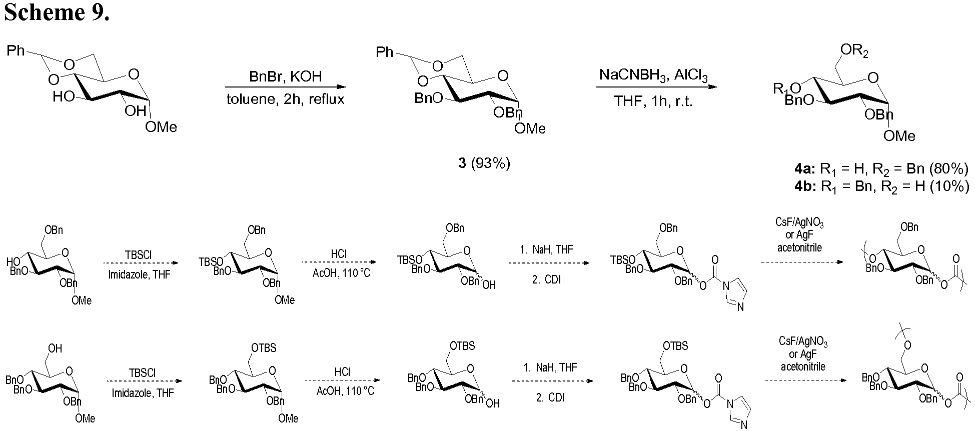

### Example-Hyperbranched Poly(D-glucose carbonate)s via ABₙ Polymerizations

AB₂, AB₃ and AB₄ monomers are possible from the five hydroxyls of D-glucose, with two, one or zero added protecting groups, respectively, which may be used to form corresponding hyperbranched poly(D-glucose carbonate)s. Shown in **Scheme 10** are the two potential AB₄ and A₄B monomers, **19** and **20**, which employ trimethylsilyl groups and chloroformates, and their corresponding hyperbranched polymers. Although each D-glucose monomer could be connected into the polymer architecture through 1, 2, 3, 4, or 5 sites (steric effects may be limiting, though, especially for 4 or 5 connections), for simplicity, only fragments of the total structures with limited branching are included in the scheme. The hydroxyl-terminated hyperbranched structures, illustrated as **21,** with the single focal point group represented as an OR group, will be the desired final polymer structure. As with the polymerizations of **17a** and **17b**, the polymerizations of **19** and **20** may not result in retention of configuration of the anomeric carbon. The stereochemistries of the polymers can be determined by NMR spectroscopy and specific rotation measurements, and then related to their physical and mechanical properties, if desired. Neither monomer **19** nor **20** requires protecting groups and each may afford a hyperbranched poly(D-glucose carbonate) that exhibits interesting properties and will undergo hydrolysis to D-glucose and carbon dioxide. The A₄B monomer, **20,** is particularly attractive due to its ability to be directly and conveniently converted into **21** under mild conditions. Removal of the TMS protecting groups in the presence of the activated carbonate linkages of **22** can be more challenging. Typical reaction conditions will be examined and optimized. Dimethylphenylsilyl groups may be substituted for the TMS groups to give a UV chromophore to assist with visualization during monitoring of the reactions and chromatographic purification. Additionally, if the TMS or DMPS groups are found to be too labile, then they may be replaced with *tert*-butyldimethylsilyl-, *tert*-butyldiphenylsilyl- or diisopropylphenylsilyl groups.

Preparation of the AB₄ monomer, **19,** is shown to involve the 1-O-allyl protecting group to facilitate its selective removal under mild conditions. Reaction of D-glucose with allyl alcohol in the presence of boron trifluoride etherate gives selective protection of the anomeric site, **23.** Installation of trimethylsilyl ethers at the remaining four hydroxyl sites, removal of the allylic protecting group and introduction of the chloroformate will give **19.** Alternatively, this last step can be replaced by reaction of the tetratrimethylsilylated-1-ol with sodium hydride and carbonyldiimidazole, to give a carbonylimidazolide if the chloroformate is found to be too unstable.

Synthesis of the A₄B monomer, **20,** relies upon preparation of the 1-O-trimethylsilylated sugar, **24.** According to literature procedures, regio- and stereoselective deacylation of the fully acetylated α- or β-D-glucose, **25,** under reaction with hydrazine in N,N-dimethylformamide or with ammonia in tetrahydrofuran/methanol affords the product with the anomeric site of a majority α-configuration, **26.** Silylation by reaction with trimethylsilyl chloride in dichloromethane containing triethylamine then gives the silylated β-anomer. Full deacetylation was conducted under mild conditions, for instance by stirring with methanol, water, and triethylamine (8:1:1) at room temperature overnight, to afford the 1-O-trimethylsilylated-β-D-glucose, **24**. The four hydroxyl groups will then be transformed to chloroformates by reaction with diphosgene, in the presence of activated carbon and N,N-dimethylaniline.

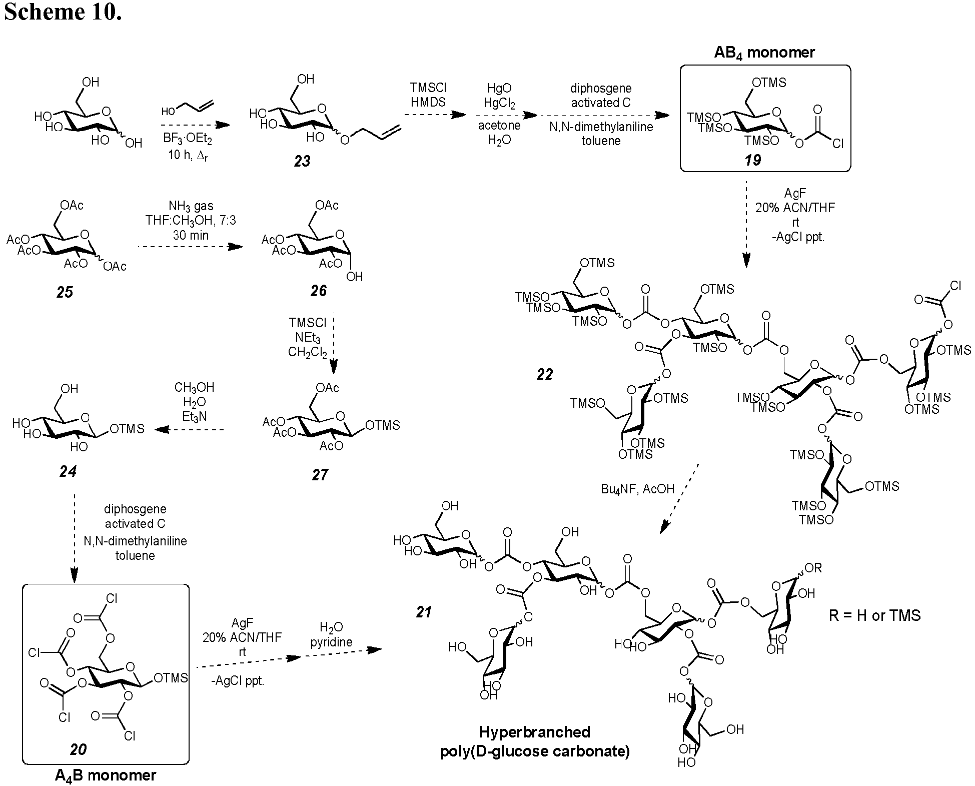

### Comparative Example-Polycarbonates from the Polyhydroxyl Natural Product Quinic Acid

*Instrumentation.* The ¹H NMR (300 and 500 MHz) and ¹³C NMR (75 and 125 MHz) spectra were obtained on either a Varian Mercury 300 MHz or Inova 500 MHz spectrometer using the solvent as internal reference. Glass transition (T_{g}) temperatures were measured by differential scanning calorimetry on a Mettler Toledo DSC822^{e} (Mettler Toledo Inc., Columbus, OH), with a heating rate of 20 °C/min. The T_{g} was taken as the midpoint of the inflection tangent, upon the third heating scan. Thermogravimetric analysis was performed under N₂ atmosphere using a Mettler Toledo model TGA/SDTA851^{e}, with a heating rate of 10 °C/min. Gel permeation chromatography (GPC) measurements were conducted on two different systems, both equipped with a Waters Chromatography, Inc. (Milford, MA) model 1515 isocratic pump, a model 2414 differential refractometer, and a three-column set of Polymer Laboratories, Inc. (Amherst, MA) Styragel columns (PL_{gel} 5 µm Mixed C, 500 Å, and 10⁴ Å, 300 × 7.5 mm columns) for the tetrahydrofuran system or a three-column set of Waters Chromatography, Inc. (Milford, MA) Styragel columns (HR 2, HR 4E and HR4, 300 × 7.8 mm columns) for the dimethylformamide system. The systems were equilibrated at 35 °C in THF or 70 °C in DMF, which served as the polymer solvent and eluent (flow rate set to 1.00 mL/min). Polymer solutions were prepared at a known concentration (ca. 3 mg/mL) and an injection volume of 200 µL was used. Data collection and analyses were performed with Precision Acquire software and Discovery 32 software, respectively (Precision Detectors). The differential refractometer was calibrated with standard polystyrene materials (SRM 706 NIST) for the tetrahydrofuran system and poly(ethylene glycol) for the dimethylformamide system. The infrared measurements were obtained with a Shimadzu IR Prestige, FTIR spectrophotometer equipped with an ATR Accessory.

*Materials.* Quinic acid **1** (Alfa Aesar, 98%), trichloromethyl chloroformate (Alfa Aesar, 98%), Amberlyst® (Sigma Aldrich, 15-ion exchange resin), dimethylaminopyridine, triethylamine (Sigma Aldrich, >99%), tert-butyldimethylsilyl chloride (Alfa Aesar, 97%) and boron trifluoride diethyl etherate (Alfa Aesar, >98%) were used as received. Pyridine was distilled over potassium hydroxide, tetrahydrofuran and dimethylformamide were dried through columns (J. C. Meyer Solvent Systems). (1R,3R,4S,5R)-5-tert-butyldimethylsilyloxy-1,4-dihydroxy-cyclohexane-1,3-carbolactone **2,** (1R,3R,4S,5R)-4-tert-butyldimethylsilyloxy-1,4-dihydroxy-cyclohexane-1,3-carbolactone **3** and calcium sulfonate resin were synthesized as described in the literature.

*General procedure of the co-polymerization between diol quinic acid 2 or 3 and trichloromethyl chloroformate:* Trichloromethyl chloroformate was added to a cold (0 °C) solution of diol quinic acid in pyridine (600 g/L) under nitrogen in three times over ca. 10 min. Caution! Trichloromethyl chloroformate is highly toxic by inhalation and ingestion, use of respiratory mask is required. After 48 h at room temperature, a saturated solution of sodium bicarbonate was added until no further emission of carbon dioxide was observed. The residue was diluted with dichloromethane; the organic layer obtained was washed with a 10% solution of hydrochloric acid, then dried with magnesium sulfate, filtered and concentrated under reduced pressure. The crude polymer was dissolved in dichloromethane followed by precipitation in cold methanol to afford the desired polymer as a white solid.

Synthesis of **4:** The general procedure for co-polymerization was applied to a solution of diol quinic acid **2** (500 mg, 1.73 mmol) in pyridine (850 µL) and trichloromethyl chloroformate (207 µL, 1.73 mmol) to obtain a white solid (400 mg, 75%). ¹ H NMR (CDCl₃, 300 MHz): δ 5.13 (bs, 1H, H-4), 4.78-4.92 (m, 1H, H-3), 4.07 (bs, 1H, H-5), 2.19-3.17 (m, 4H, H-2, H-6), 0.83-0.92 (m, 9H, (CH₃)₃CSi), 0.04-0.09 (m, 6H, (CH₃)₂Si). ¹³C NMR (CDCl₃, 75 MHz): δ 170.5-170.8 (CO-lactone), 150.0-153.3 (CO-carbonate), 78.1-78.4 (C1), 73.3-73.7 (C3), 70.5-71.7 (C4), 65.6-65.8 (C5), 36.9-37.1 (C6), 33.2-34.0 (C2), 25.4-25.6 ((CH₃)₃CSi), 17.7-18.0 ((CH₃)₃CSi), ⁻5.3-⁻5.0 ((CH₃)₂Si). IR vₘₐₓ/cm⁻¹: 3104-2758, 1803, 1746, 1240, 124, 1033, 843, 777. Tg = 209 °C. (T_{d})ₒₙₛₑₜ = 284 °C; (T_{d})₅₀ = 384 °C; 284-420 °C, 89% mass loss; 11% mass remaining at 500 °C.

Synthesis of **5**: The general procedure for co-polymerization was applied to a solution of diol quininc acid **3** (500 mg, 1.73 mmol) in pyridine (850 µL) and trichloromethyl chloroformate (207 µL, 1.73 mmol) to obtain a white solid (462 mg, 85%). ¹ H NMR (CDCl₃, 300 MHz): δ 4.76-4.77 (m, 1H, H-5), 4.67 (bs, 1H, H-3), 4.37 (bs, 1H, H-4), 3.00-3.15 (m, 1H, H-2), 2.62-2.69 (m, 1H, H-2), 2.26-2.34 (m, 2H, H-6), 0.83-0.92 (m, 9H, (CH₃)₃CSi), 0.04-0.09 (m, 6H, (CH₃)₂Si). ¹³C NMR (CDCl₃, 75 MHz): δ 170.6-170.7 (CO-lactone), 149.7-152.8 (CO-carbonate), 78.1-78.5 (C1), 76.1-76.4 (C3), 71.9-72.74 (C5), 64.5 (C4), 32.7-33.0 (C2, C6), 25.5 ((CH₃)₃CSi), 17.9 ((CH₃)₃CSi), -5.1 ((CH₃)₂Si). IR vₘₐₓ/cm⁻¹: 3096-2272, 1803, 1746, 1227, 1060, 832, 776. T_{g} = 225 °C. (T_{d})ₒₙₛₑₜ = 302 °C, (T_{d})₅₀ = 373 °C; 302-416 °C, 92% mass loss; 8% mass remaining at 500 °C.

*General procedure of the removal of the TBS-protecting group:* Boron trifluoride diethyl etherate was added to a solution of protected poly(quinic acid carbonate) in anhydrous tetrahydrofuran at different temperatures. After different durations, Amberlyst® H⁺ (15 x mass of protected poly(quinic acid carbonate)) and calcium sulfonate resin (15 x mass of protected poly(quinic acid carbonate)) were added to the reaction and the mixture was stirred 2 h at the same temperature as the reaction occurred. The resins were removed by filtration and rinsed twice with tetrahydrofuran. The filtrate was concentrated under reduced pressure, and then the residue was triturated with methanol to afford a white precipitate, which was collected by filtration and dried under vacuum pump.

Synthesis of **6**: The general procedure for the removal of the TBS-protecting group was applied to a solution of polymer **4** (30 mg, 0.1 mmol) in anhydrous tetrahdrofuran (3 mL) and boron trifluoride diethyl etherate (60 µL, 0.5 mmol) to obtain a white solid (18.3 mg, 74%). ¹H NMR (DMSO-d₆, 500 MHz): δ 4.96-5.20 (m, 2H, H-3, H-4), 4.02-4.06 (m, 1H, H-5), 2.95-2.97 (m, 1H, H-2), 2.55-2.61 (m, 1H, H-2), 2.09-2.44 (m, 2H, H-6), 0.83-0.87 (m, 3.6H, (CH₃)₃CSi), 0.05-0.07 (m, 2.5H, (CH₃)₂Si). IR vₘₐₓ/cm⁻¹: 3226-3050, 3022-2774, 1810, 1760, 1223, 1120, 1026, 839, 775. T_{g} = 230 °C_{.} (T_{d})ₒₙₛₑₜ = 334 °C, (T_{d})₅₀ = 395 °C; 334-430 °C, 93% mass loss; 7% mass remaining at 500 °C

Synthesis of **7**: The general procedure for the removal of the TBS-protecting group was applied to a solution of polymer **5** (30 mg, 0.1 mmol) in anhydrous tetrahydrofuran (3 mL) and boron trifluoride diethyl etherate (240 µL, 2.0 mmol) to obtain a white solid (18.0 mg, 78%). ¹H NMR (DMSO-d₆, 500 MHz): δ 4.95-4.97 (m, 1H, H-5), 4.57-4.62 (m, 1H, H-3), 4.39-4.44 (m, 1H, H-4), 2.89-3.12 (m, 4H, H-2, H6), 0.86-0.87 (m, 2.4H, (CH₃)₃CSi), 0.03-0.08 (m, 1.6H, (CH₃)₂Si). IR vₘₐₓ/cm⁻¹: 3351-3084, 3039-2739, 1812, 1759, 1231, 1067, 834, 780. Tg = 250 °C. (T_{d})ₒₙₛₑₜ = 320 °C, (T_{d})₅₀ = 397 °C; 320-445 °C, 82% mass loss; 18% mass remaining at 500 °C.

### Results and Discussion

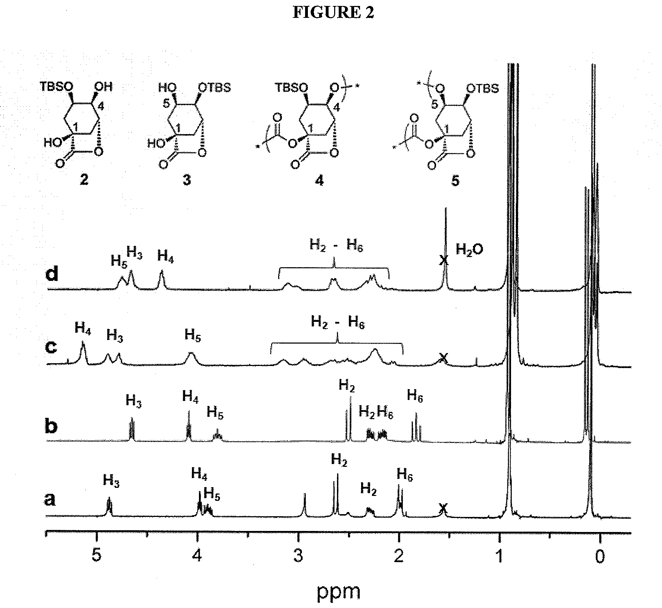

Beginning from quinic acid, **1,** the regioisomeric monomers having 1,4- and 1,5-diols, **2** and **3,** respectively, were prepared by a two-step reaction sequence (Scheme 11: Syntheses of diol monomers 2 and 3 and poly(quinic acid carbonate)s 4 and 5). Quinic acid **1** underwent lactonization between positions 1 and 3 with 75% yield. The procedure for silylation of lactonized quinic acid described by Garg, N., K.; Caspi, D. D.; Stoltz, B. M. J. Am. Chem. Soc. 2004, 126, 9552-9553 was then performed with modification of the duration and the temperature (overnight at room temperature, instead of 2 h at - 15 °C) to give diol monomers **2** and **3** with 56 and 24% yields, respectively, isolated by column chromatography. The chemical shifts of the protons from the quinic acid moiety (Figure 2, a and b) confirmed the structures of diols **2** and **3,** as described previously.

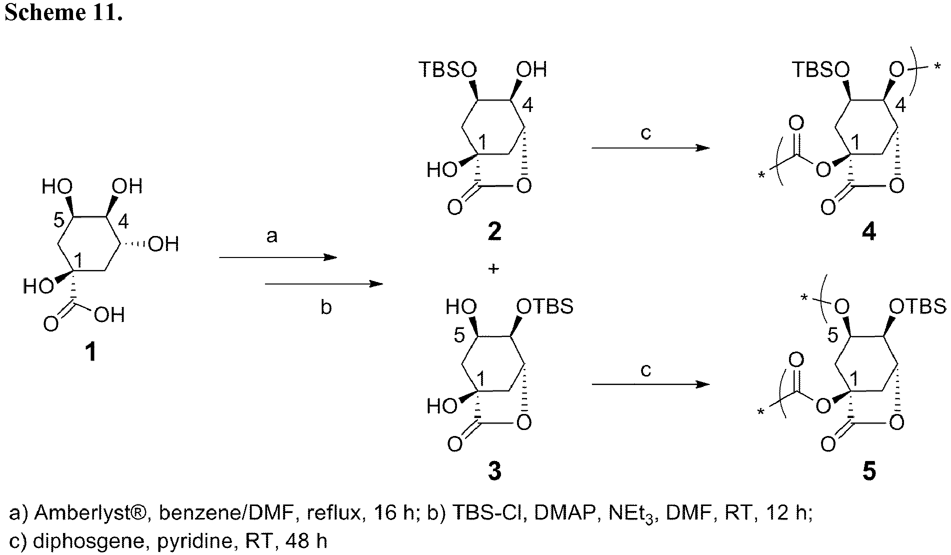

The co-polymerizations between diol quinic acid **2** and phosgene generated by different carbonylation agents were tested under a variety of reaction conditions. After screening the co-polymerizations of **2** with phosgene in toluene/pyridine, diphosgene in pyridine, triphosgene in pyridine, and carbonyldiimidazole in pyridine, each with and without added solvents, at various temperatures and concentrations over *ca.* twenty different reactions, it was observed by SEC (using tetrahydrofuran as the eluent and with polystyrene calibration standards) that only trichloromethyl chloroformate (diphosgene) in the presence of pyridine (being the base as well as the solvent) allowed for the formation of polycarbonates.

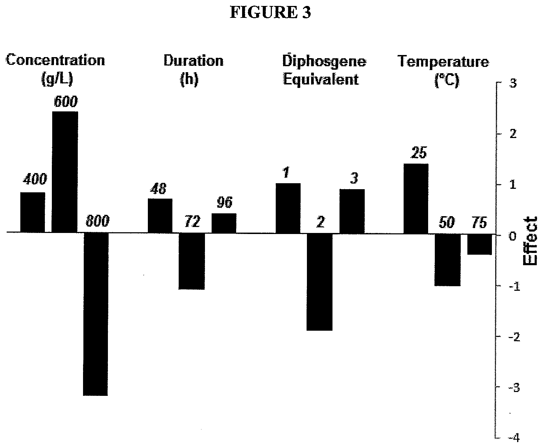

The temperature, duration, concentration and number of equivalents of diphosgene were then optimized by an experimental design (matrix 4³ where 4 is the number of factors and 3 the number of levels tested for each factor) in order to identify with only nine experiments the conditions that gave rise to polycarbonates having the largest molecular weights with the highest monomer conversions (Table 1, entries 1-9). The average and the effect of each level, for all the factors, were calculated: the concentration (600 g/L) was identified as the most positive factor for this reaction (Figure 3). Using the highest positive levels of each of the factors, the experimental design was validated to give poly(1,4-quinic acid carbonate) 4 with 75% yield after precipitation showing the best results with 99% conversion of the diol quinic acid 2 and a peak average molecular weight of 9.7 kDa (Table 1, entry 10). However, the lack of solubility of the polymer in pyridine (solid formation occurred 30 min after the end of diphosgene addition) was observed, questioning the necessity to perform the polymerization over 48 h. The results of the experiments quenched after 30 min and 24 h (Table 1, entries 11 and 12) showed that even if the mixture became non-homogeneous, the reaction continued to proceed, and that the duration was required to allow the growth of the polymer.

**Table 1. Conditions and results of the experimental design for the co-polymerizations between diol quinic acid 2 and trichloromethyl chloroformate.**

| **Entry** | **Concentration (g/L)^{a}** | **Duration (h)** | **Eq.^{b}** | **Temperature (°C)** | **Mₚ (kDa)^{c}** | **Conversion (%)^{d}** | **Yield^{e}** |
|---|---|---|---|---|---|---|---|
| 1 | 400 | 48 | 1 | 25 | 8.2 | 99 | 62 |
| 2 | 400 | 72 | 2 | 50 | 1.1 | 47 | - |
| 3 | 400 | 96 | 3 | 75 | 6.1 | 95 | 43 |
| 4 | 600 | 48 | 2 | 75 | 5.1 | 97 | 40 |
| 5 | 600 | 72 | 3 | 25 | 8.0 | 96 | 51 |
| 6 | 600 | 96 | 1 | 50 | 7.2 | 99 | 51 |
| 7 | 800 | 48 | 3 | 50 | 1.8 | 94 | 51 |
| 8 | 800 | 72 | 1 | 75 | 0.7 | 40 | - |
| 9 | 800 | 96 | 2 | 25 | 1.1 | 96 | 13 |
| 10 | 600 | 48 | 1 | 25 | 9.7 | 99 | 75 |
| 11 | 600 | 0.5 | 1 | 25 | 1.4 | 10 | 70 |
| 12 | 600 | 24 | 1 | 25 | 3.1 | 90 | 7 |

| | | | | | | | |
|---|---|---|---|---|---|---|---|
| ^{a}Concentration of **2** in distilled pyridine. ^{b}Number of equivalents of trichloromethyl chloroformate vs. **2.** ^{c}Peak average molecular weight determined by SEC-THF for crude polymers vs. polystyrene standards. ^{d}Determined by ¹H NMR spectroscopy by dividing the value of the integration at 5.13 ppm (H4 polymer) and the integration between 4.78 and 4.92 ppm (H3 monomer + polymer). ^{e}After precipitation in cold methanol. | | | | | | | |

The optimized experimental conditions for the polymerization of **2** were then applied to the 1,5-diol quinic acid monomer **3** to afford poly(1,5-quinic acid carbonate) **5.** The co-polymerizations of the diol monomers **2** and **3** with phosgene to give polycarbonates **4** and **5** were clearly demonstrated by ¹H NMR spectroscopy (Figure 2a *vs*. 2c and Figure 2b vs. 2d for the monomers and corresponding polymers, respectively) by observation of the downfield shifts for most protons from the quinic acid moiety and, particularly, the significant downfield shift (1-1.2 ppm) of the protons on the carbons involved in the formation of the carbonate linkages; H-4 for **4** and H-5 for **5.** The carbonate linkages were directly observed by the introduction of ¹³C NMR resonances at *ca.* 150 ppm, in addition to the lactone ¹³C signal at *ca.* 170 ppm. The presence of the carbonate and lactone functionalities was confirmed additionally by two C=O absorbance bands at 1746 and 1803 cm⁻¹ in the IR spectra, whereas the monomer exhibited only a single absorbance at 1777 cm⁻¹. Although the molecular weight distribution of **5** was slightly broader than that of **4,** as determined by GPC, the two regioisomeric polycarbonates had similar molecular weights (Table **2,** entries 1 and 2), which allowed for direct comparisons of the properties of the materials in relation to the regiochemistry.

**Table 2. Properties of protected poly(quinic acid carbonate)s 4 and 5 obtained by co-polymerization between trichloromethyl chloroformate and diols 2 and 3.**

| **Entry** | **Polymer** | **Yield (%)^{a}** | **Mₙ (kDa)^{b}** | **PDI^{b}** | ***T*_{g} (°C)** | **(*T*_{d})ₒₙₛₑₜ^{c} (°C)** | **(T_{d})₅₀^{d} (°C)** |
|---|---|---|---|---|---|---|---|
| **1** | **4** | 75 | 7.5 | 2.0 | 209 | 284 | 331 |
| **2** | **5** | 85 | 7.7 | 3.5 | 225 | 302 | 373 |

| | | | | | | | |
|---|---|---|---|---|---|---|---|
| ^{a}After precipitation in cold methanol. ^{b}Determined by SEC-THF for precipitated polymers vs. polystyrene standards. ^{c}Temperature degradation onset observed by TGA. ^{d}Temperature at 50 wt. % loss observed by TGA. | | | | | | | |

The poly(quinic acid)s exhibited thermal properties in agreement with other highly rigid polycarbonate materials. The onset temperatures for thermal decomposition for both polycarbonates (between 284 and 302 °C) were comparable to the typical range of decomposition temperatures of polycarbonates from 200 °C (polyethylene carbonate) to 350 °C (poly(bisphenol A carbonate)). High glass transition temperatures (above 205 °C) have been observed for these polymers exceeding that of commercial poly(bisphenol A carbonate) by *ca.* 50 °C. An absence of melting transitions (Tₘ) or crystallization transitions (T_{c}), below the thermal decomposition onset temperatures, suggested that the polymers were amorphous. These exceptional thermal characteristics may be due to the presence of the bicyclic backbone in these materials, which introduces rigidity to the polymer similar to the cyclic backbone of poly(cyclohexene carbonate) that shows a 90 °C increase in *T*_{g} in compared to its non-cyclic analog poly(ethylene carbonate). Moreover, monomer regiochemistry showed a significant impact on the polycarbonate *T*_{g} (20 °C difference between **4** and **5**), which suggests that the O-1, O-4 carbonate linkage of **4** is more flexible than its regioisomer, **5,** linked between O-1, O-5.

Removal of the silyl protecting groups was performed in mild conditions to conserve the carbonate and lactone linkages. Although the usual fluoride reagents, including tetrabutylammonium fluoride, hydrofluoric acid with pyridine, in situ generation of hydrofluoric acid by reaction of potassium fluoride with trimethylsilylchloride, were unsuccessful in selective removal of the protecting groups, surprisingly boron trifluoride diethyl etherate (BF₃·Et₂O)²⁵ allowed partial removal of the protecting groups without causing degradation of the lactone and carbonate links (Scheme 12: Partial removal of the TBS protecting groups of polycarbonates 4 and 5). The deprotection of **5** was performed at room temperature with 20 equivalents of BF₃·Et₂O, whereas a drastic reduction of molecular weight and coincident effects on the thermal properties was observed when compound **4** underwent the deprotection at room temperature with only 5 equivalents of the BF₃·Et₂O reagent (Table 3, entry 6). To quench the reaction and remove the silyl alcohol generated, calcium sulfonate resin combined with sulfonic acid resin was used, as described by Parlow *et al* for quenching and purification of desilylating reactions involving tetrabutylammonium fluoride as reagent. (Parlow, J. J.; Vazquez, M. L.; Flynn, D. L. Bioorg. Med. Chem. Lett. 1998, 8, 2391-2394.) The complete deprotection of both of the materials was not achieved, however, without degradation of the polymer, as observed by a decrease in the glass transition temperatures and the thermal stabilities; the maximum TBS removal observed leading to poly(quinic acid carbonate)s **6** and **7** was 59 and 73%, respectively (Table 3).

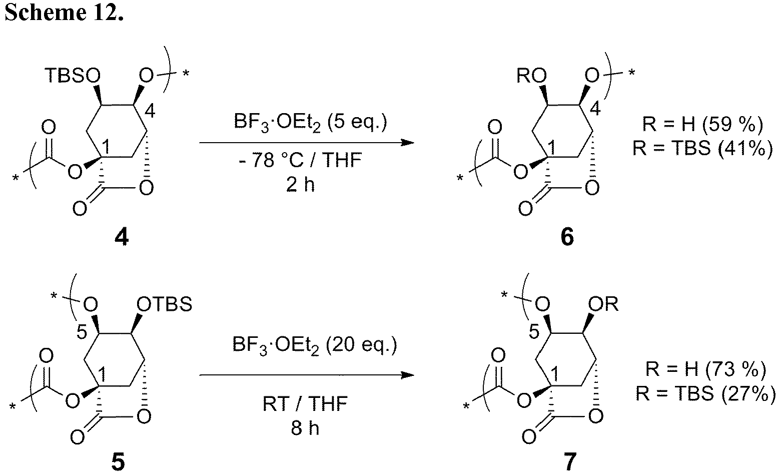

**Table 3. Conditions and results of the TBS removal of 4 and 5**

| **Entry** | **Polymer** | **Eq.^{a}** | **Duration (h)** | **Temperature (°C)** | **Removal TBS (%)^{b}** | **M*ₙ* (kDa)^{c}** | **PDI^{c}** | ***T_{g}* (°C)** | **(*T*_{d})ₒₙₛₑₜ^{d} (°C)** |
|---|---|---|---|---|---|---|---|---|---|
| **1** | **4** | 5 | 1 | -78 | 28 | 4.4 | 3.1 | 214 | 328 |
| **2** | **4** | 5 | 2 | -78 | 59 | 3.3 | 2.1 | 230 | 334 |
| **3** | **4** | 5 | 4 | -78 | 46 | 4.9 | 2.1 | 228 | 316 |
| **4** | **4** | 5 | 6 | -78 | 44 | 3.8 | 1.9 | 212 | 325 |
| **6** | **4** | 5 | 15 | RT | 90 | 0.4 | 2 | 70 | 233 |
| **7** | **5** | 20 | 1.5 | RT | 44 | 8.4 | 2.9 | 235 | 316 |
| **8** | **5** | 20 | 3 | RT | 46 | 8.8 | 3.7 | 238 | 319 |
| **9** | **5** | 20 | 6 | RT | 55 | NS^{e} | NS^{e} | 240 | 320 |
| **10** | **5** | 20 | 8 | RT | 73 | NS^{e} | NS^{e} | 250 | 320 |
| **11** | **5** | 20 | 15 | RT | 63 | NS^{e} | NS^{e} | 244 | 317^{f} |
| **12** | **5** | 20 | 24 | RT | 49 | 7.3 | 2.6 | 216 | 310^{f} |

| | | | | | | | | | |
|---|---|---|---|---|---|---|---|---|---|
| ^{a}Number of equivalents of trifluoride diethyl etherate vs. **4** or **5.** ^{b}Determined by ¹H NMR spectroscopy (DMSO-d₆). ^{c}Determined by SEC-DMF vs. poly(ethylene glycol) for precipitated polymers. ^{d}Temperature degradation onset observed by TGA. ^{e}Not soluble in DMF. ^{f}Observation of a loss of 20% mass between 90 - 180 °C, due to loss of water. | | | | | | | | | |

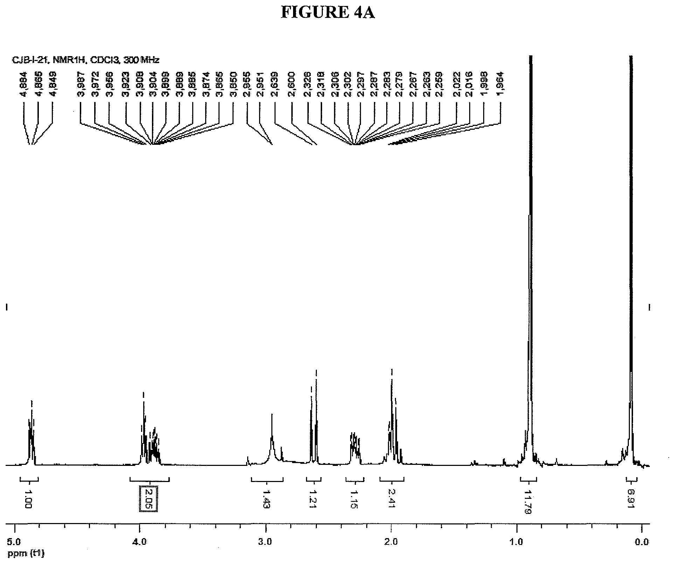

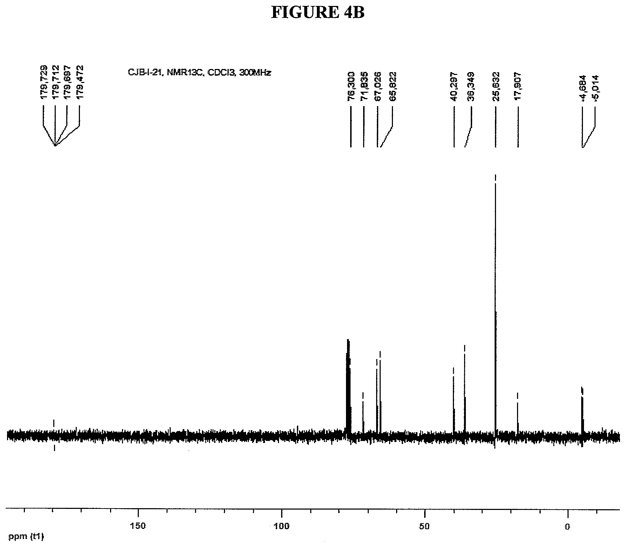

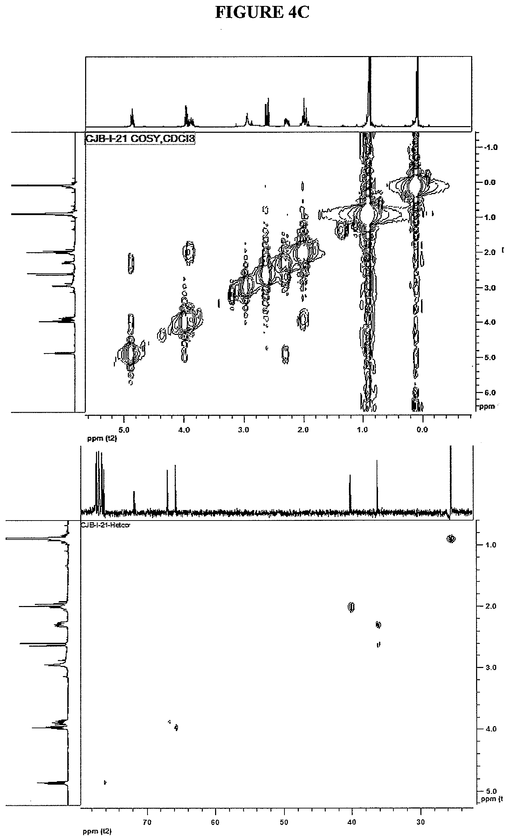

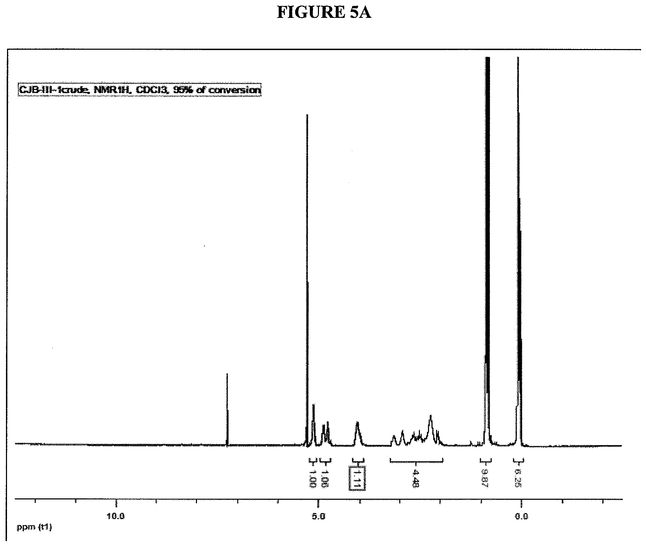

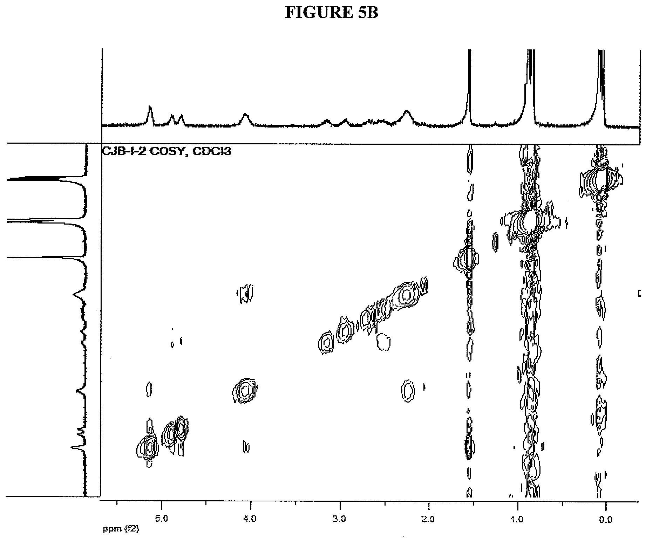

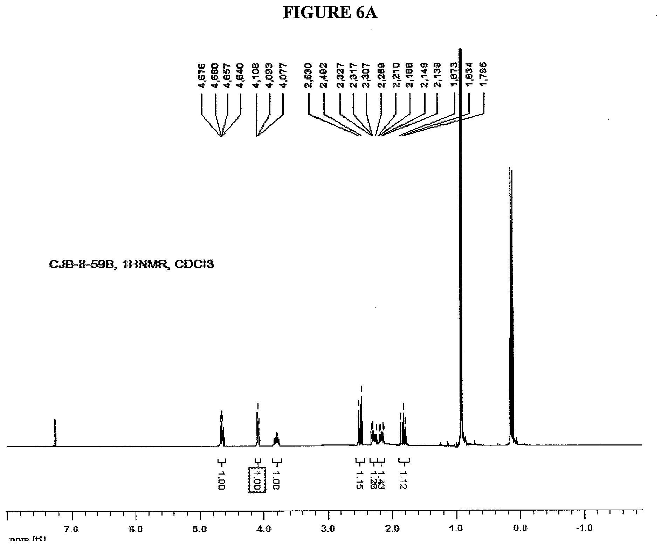

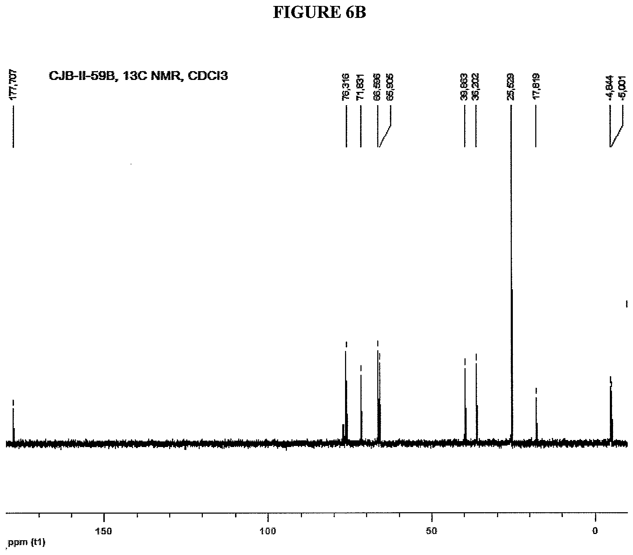

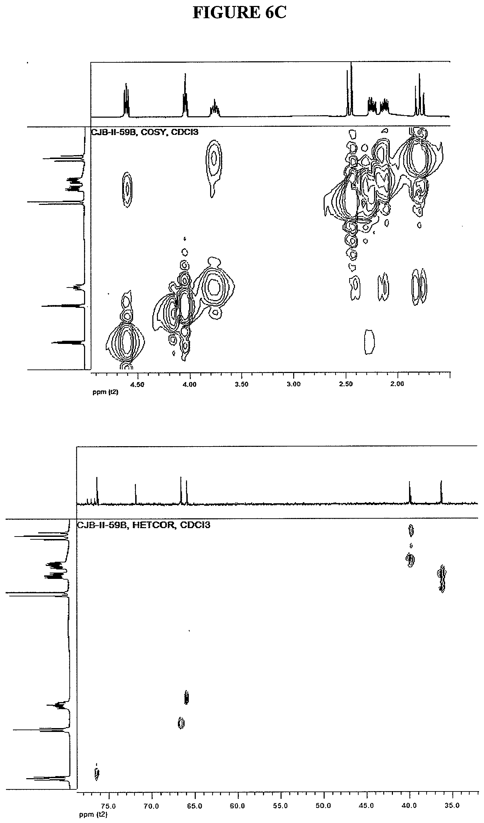

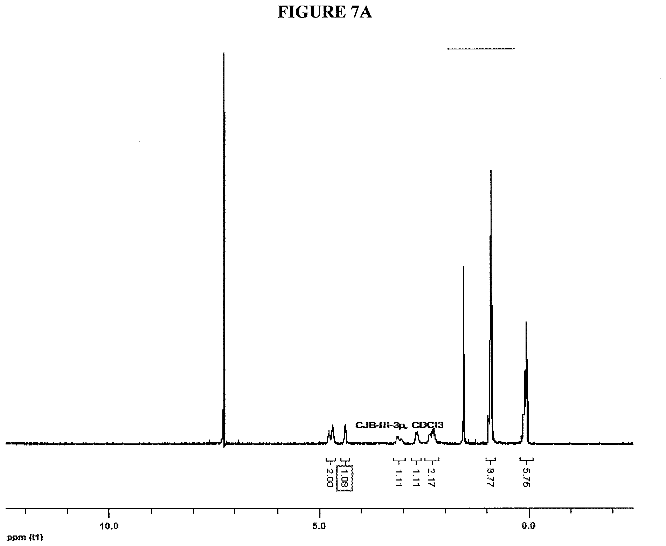

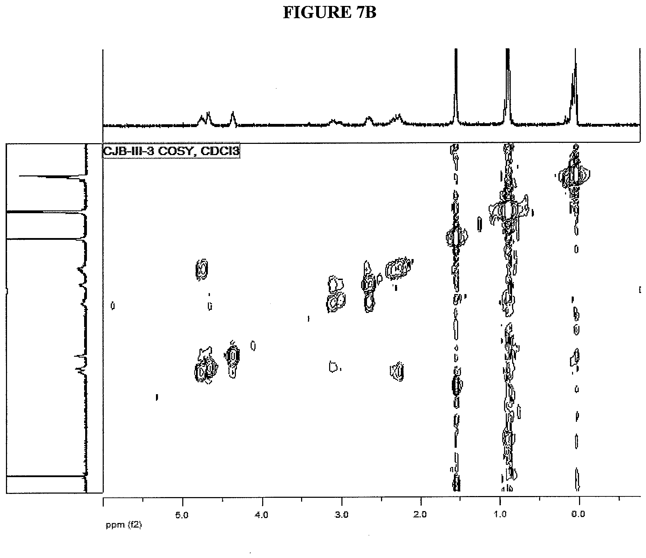

Significant changes in properties were observed upon removal of even a fraction of the TBS protecting groups. Each regioisomer underwent a 10-20 °C increase in *T*_{g}, which can be reasonably attributed to hydrogen-bond network formation. The presence of hydroxyl groups, esters and carbonates along the backbone and, together with maintenance of the rigid bicyclic monomer unit backbone structure, could explain the high glass transition temperatures observed at 230 °C and 250 °C for poly(quinic acid carbonate)s **6** and **7,** respectively (Table 3, entries 2 and 10). As the extent of deprotection increased, the solubility decreased. Ultimately, at long deprotection reaction times, the solubility increased and the *T*_{g} decreased, due to significant degradation of the polymers.
¹H, ¹³C and 2D (COSY and Hetcor) NMR spectra for the monomers and polymers are shown in Figures 4-7.

### Conclusions

Unique and degradable, polycarbonates, starting from a renewable resource, quinic acid, have been synthesized by condensation polymerization with phosgene generated *in situ* from trichloromethyl chloroformate. The conditions of polymerization have been tuned to result in the generation of *tert*-butyldimethylsilyloxypoly(quinic acid carbonate)s having relatively high molar masses (Mₙ = 7.5-7.7 kDa) and exhibiting simultaneously high glass transition temperatures (*T*_{g} = 209-229 °C) and good resistance to thermal degradation ((*T*_{d})ₒₙₛₑₜ = 284-302 °C) compared to previously reported polycarbonates. Obviously, monomer regiochemistry has been identified as a crucial parameter that contributes to the thermal properties of the polymers with a 20 °C difference in *T*_{g} between each of the regioisomers, and in terms of reactivity during removal of the protecting group with initial degradation observed after 8 h at room temperature when carbonate is linked on O-1, O-5, against less than 4 h at low temperature when the linkage was created between O-1, O-4. The study of the TBS removal encountered a competition between deprotection and degradation of the polymer with a limitation at 60 - 70% of removal of the protecting groups. Even with the limited deprotection, the glass transition temperatures increased by 10-20 °C (*T*_{g} = 230-250 °C), which is beginning to approach the onset of thermal degradation, and the solubility decreased. These polycarbonates may find application as replacements for poly(bisphenol A carbonate) and/or as orthopedic tissue engineering materials, due to their rigid bicyclic backbone structure and aliphatic nature that will allow for the breaking down of the polymer to carbon dioxide and polyhydroxyl bioresorbable degradation products.

### Comparative Example-Linear Poly(quinic acid carbonate)s via Ring Opening Polymerizations

The preparation of poly(4,5-quinic acid lactone carbonate)s by ring opening polymerization also may be used (e.g., **Scheme 13**). As with AB polymerizations, ring opening polymerization avoids complications related to stoichiometric equivalencies of functionalities. The 1,3-lactone and 4,5-carbonate of quinic acid, **36,** has been prepared in good yield, and was studied for ring opening polymerization after a simple protection of the remaining hydroxyl group. 1,8-diazabicyclo[5.4.0]undec-7-ene (DBU) has been reported as an efficient catalyst for the ring opening polymerization of a five-membered cyclic carbonate, methyl 4,6-O-benzylidene-2,3-O-carbonyl-α-D-glucopyranoside, without elimination of carbon dioxide, so DBU was studied initially as the polymerization catalyst. Benzyl alcohol, as an initiator, will give unique proton resonances for assistance in characterization of the polymers, including calculation of the average degrees of polymerization. DBU has been found to be effective for ring opening polymerization of six-membered cyclic carbonates. More recently, investigators have polymerized six-membered cyclic trimethylene carbonate derivatives, including those carrying a sugar side chain, using DBU with an added thiourea co-catalyst. If necessary, such conditions can be employed. Finally, deprotection will afford 37, which will be characterized for composition, structure, properties and degradability. Molecular models of the poly(quinic acid carbonates) suggest that the 4,5-regioisomer, **37,** will be more sterically-hindered and less able to adopt conformations that allow for good inter-chain packing than will be the 1,4-regioisomer, **29.** Therefore, **37** is expected to have poorer mechanical properties than will **29.**

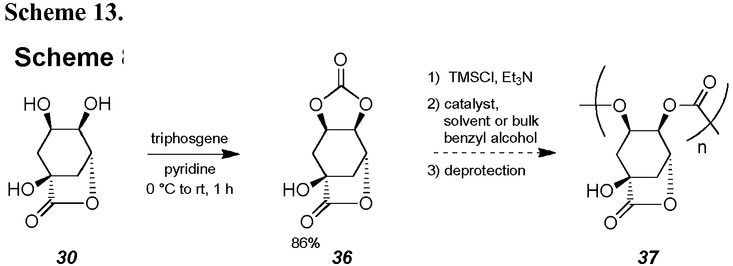

### Comparative Example-Linear Poly(quinic acid carbonate)s via AB Polymerizations

Linear AB condensation polymerizations may be used for access to the poly(1,4-quinic acid carbonate)s, **29** (**Scheme 14**) In this case, the (2-nitrophenyl)acetyl group was used to protect the 5-hydroxyl, as the tert-butyldimethylsilyl ether of would undergo reaction with silver fluoride. From **33,** introduction of the trimethylsilyl protecting group lead to a mixture of products, from which chromatographic separation allowed for isolation of monosilylated intermediate **38.** Introduction of the chloroformate then affords the AB monomer. Polymerization conditions were investigated and optimized, starting from conditions that have worked well for us in the past. Finally, deprotection affords **29.**

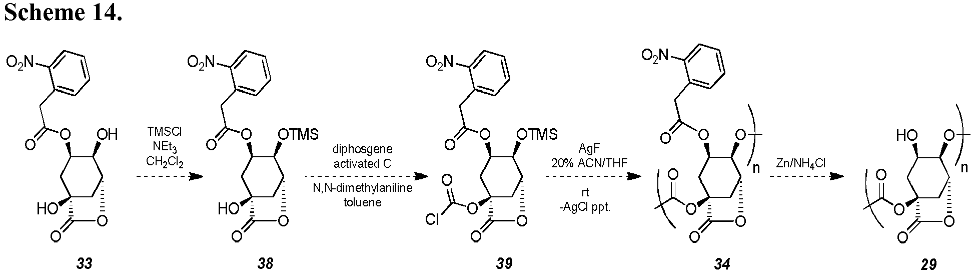

### Comparative Example-Hyperbranched Poly(quinic acid carbonate)s via A₂B Polymerization

The monosilylated lactone of quinic acid, 2, in addition to serving as a diol for direct AA + BB polycondensation (**Scheme 11**), offers an opportunity to prepare an A₂B monomer, **41**, leading to hyperbranched poly(quinic acid carbonate)s (**Scheme 15**). With each repeat unit having a rigid bicyclic framework and the linkages between the repeat units involving only a carbonyl, the physical and mechanical properties should be of significant interest. For instance, a densely-branching architecture should limit entanglements, resulting in low modulus, strength and toughness in the bulk and low viscosity in solution, whereas the intermolecular hydrogen bonding through the hydroxyl, ester and carbonate units should increase the intermolecular attractive interactions, giving the opposite effects and decreasing the fluid flow behavior in the bulk. These, as well as the composition, structure, and degradation properties can be determined and correlated. To characterize the degree of branching, a technique can be employed involving silylation-based capping of the hydroxyl chain ends, followed by reduction of the polycarbonate backbone, to measure the relative numbers of dihydroxyl chain ends, mono-hydroxyl linear repeat units and non-hydroxyl branching units within **41.** The chain ends and branching units each contribute to the degree of branching.

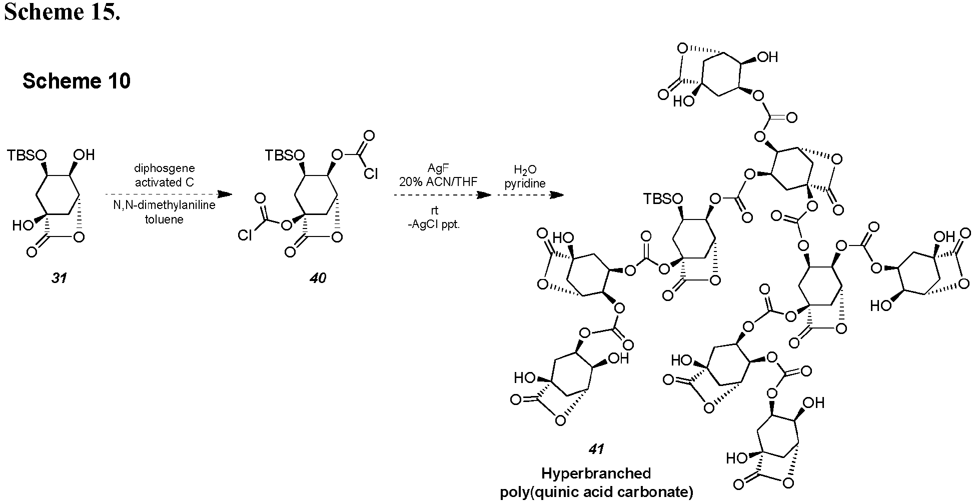

### Comparative Example-Quercetin AB linear polymerization

An example scheme for AB linear polymerization of quercetin and quercetin monomers is shown in **Scheme 16.** Deprotection would then form the polycarbonate that would release quercetin and carbon dioxide upon hydrolytic degradation.

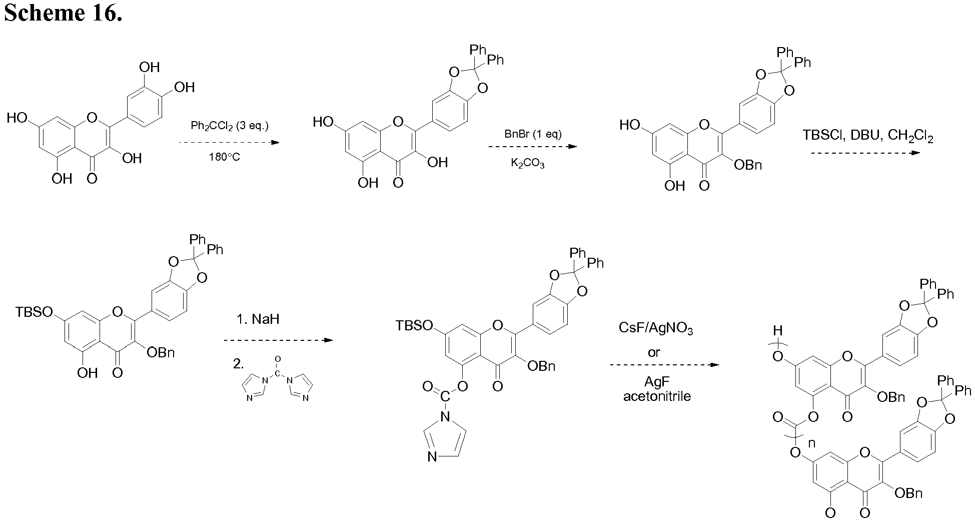

### Comparative Example-Quinic acid polycarbonate crosslinking

There are at least two general strategies for the formation of crosslinked polycarbonates based upon polyhdroxyl monomers. One involves the initial formation of (linear or branched) polymers that contain reactive sites through which crosslinks can be established. The other involves a direct crosslinking by reaction of multi-functional small molecules. Each strategy is illustrated with multiple pathways of chemistry being shown below (**Scheme 17**) for a single polyhydroxyl monomer, quinic acid.

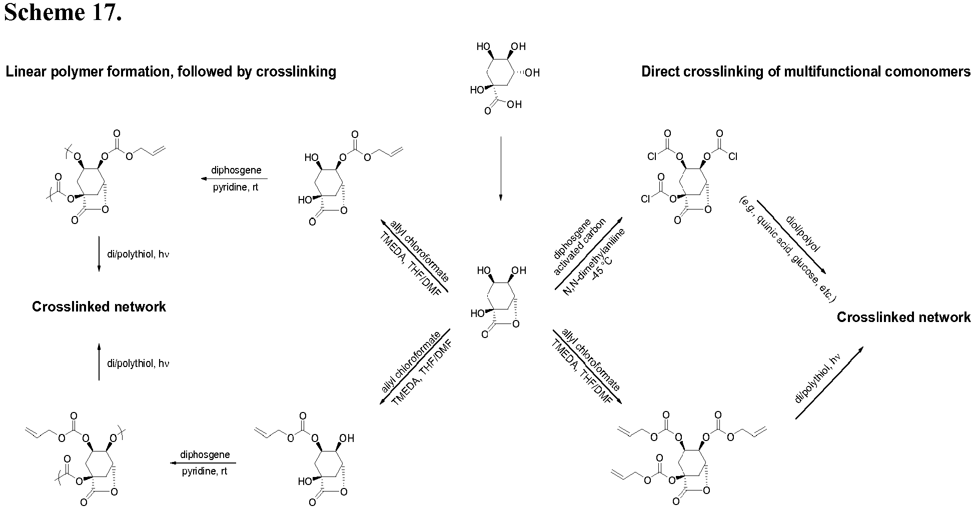

***Quinic acid polycarbonate crosslinked from linear polymer formation, followed by crosslinking: Post-polymerization photo-initiated crosslinked thiol-ene thermosets**.* 1g of thermoplastic poly(1,4-quinic acid) carbonate with pendant allyl groups in the side chain made by method described above with an allyl protecting group in place of the TBS group of the example, was dissolved in 3g choloroform in a 40 mL glass vial. A stoichiometric equivalent (1 SH:C=C) of pentaerythritol tetrakis(3-mercaptopropionate) (PETMP), and a 1 wt% equivalent of % 2,2'-dimethoxy-2-phenylacetophenone (DMPA) were added to a 40 mL glass vial. The mixture was vortexed for 15 min and allowed to sit for 1h, after which all components were completely dissolved. The solution was poured into a 2"x2" polypropylene dish and allowed to sit in a fume hood for 24 h, after which the majority of the chloroform had evaporated. The resulting film was a thermoplastic polycarbonate with PETMP and DMPA dissolved in it. The polypropylene dish was placed in a UVP CL-1000L 365 nm UV crosslinking chamber and irradiated for 30 min, after which the cured thermoset was placed under vacuum at 80°C for 5 h to remove any residual solvent. The films were then placed on Teflon sheets and post-cured under vacuum at 150 °C for 1 h. The resulting thermoset polymer film was transparent and was of appropriate sample dimensions and sufficient mechanical integrity to be machined and subjected to numerous mechanical characterization experiments, including dynamic mechanical analysis and uniaxial tensile testing experiments.

### Quinic acid polycarbonate crosslinked from direct crosslinking of multi-functional comonomers: Direct photo-initiated crosslinked thiol-ene thermosets.

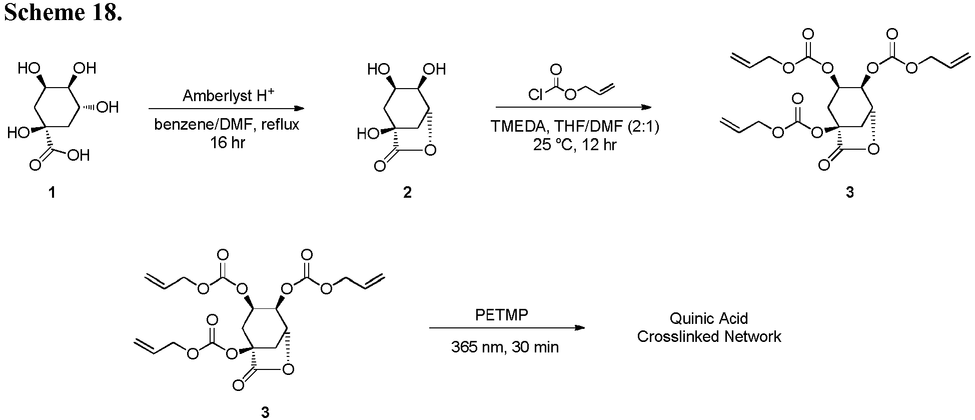

*Formation of **3** in Scheme 18.* QA lactone (**2**) (5.0448 g, 28.968 mmol) and tetramethylethylenediamine (TMEDA) (13.9470 g, 119.98 mmol)were dissolved in a 1:2 dry THF/DMF solution (30 mL) and cooled to -5° C. Allyl chloroformate (22.0 mL, 206 mmol) was added over 1.5 h and allowed to stir for 2 h at -10 to -5° C. The solution was allowed to warm to room temperature and stir for an additional 12 h. The reaction mixture was diluted with dichloromethane, filtered to remove solids, and washed with 10% HCl solution to remove residual allyl chloroformate, TMEDA, and DMF. The organic layer was dried with MgSO₄, concentrated under reduced pressure, and purified by column chromatography over silica gel (85:15 Hex/EtOAc) resulting in a clear oil (5.49 g, 44.5%).

¹H NMR (CDCl₃, 300 MHz): δ 5.98-5.83 (m, 3H, H-10), 5.41-5.25 (m, 6H, H-11), 5.34-5.33 (t, 1H, H-4), 5.06-4.98 (m, 1H, H-5), 4.9 (q, 1H, H-3), 4.64-4.60 (m, 6H, H-9), 3.21-3.14 (m, 1H, H-2eq), 2.56-2.52 (d, 1H, H-2ax), 2.46-2.38 (m, 1H, H-6eq), 2.37-2.33 (t, 1H, H-6ax). 13C NMR (CDC13, 300 MHz): δ 170.44 (C-7, CO-lactone), 153.69 (CO-carbonate), 153.22 (CO-carbonate), 152.15 (CO-carbonate), 131.06 (CH₂=C), 130.83 (CH₂=C), 130.68 (CH₂=C), 119.70 (CH=CH₂), 119.68 (CH=CH₂), 119.27 (CH=CH₂), 77.47 (C1), 73.25 (C3), 69.44-69.13(C5), 67.70 (C4), 33.30 (C2), 33.26 (C6).

*Bulk curing of quinic acid-based thiol-ene thermoset.* 4 g of triallyl carbonate quinic acid, **3**, was massed in a 40 mL glass vial, and a stoichiometric equivalent (1 SH:C=C) of pentaerythritol tetrakis(3-mercaptopropionate) (PETMP) was added to a separate vial. One wt% 2,2'-dimethoxy-2-phenylacetophenone (DMPA, wt% of total monomer mass) was added to the PETMP, vortexed for 2 min, and sonicated for 30 min at 25°C, after which the DMPA was completely dissolved in the PETMP. Compound **3** was then added to the PETMP/DMPA solution, vortexed for 2 min, and sonicated for 1 min. The quinic acid and PETMP/DMPA solution were mutually miscible, and after sitting for 45 min at 25°C, all bubbles were gone from the transparent, viscous monomer mixture. The glass vial containing the monomer mixture was placed in a UVP CL-1000L 365 nm UV crosslinking chamber and irradiated for 30 min, after which the cured thermoset was post-cured at 150°C for 1h under vacuum. The glass vial was carefully broken, and the resulting polymer sample was a transparent polymer plug, which was of appropriate sample dimensions and sufficient mechanical integrity to be machined and subjected to numerous mechanical characterization experiments, including dynamic mechanical analysis and uniaxial tensile testing experiments.

*Solution curing of quinic acid-based thiol-ene thermoset.* 1.5g of triallyl carbonate quinic acid, **3,** a stoichiometric equivalent (1 SH:C=C) of pentaerythritol tetrakis(3-mercaptopropionate) (PETMP), and a 1 wt% equivalent of % 2,2'-Dimethoxy-2-phenylacetophenone (DMPA) were added to a 40 mL glass vial. Dichloromethane was added in a 2.5:1 weight ratio of solvent:total monomer mass. Both monomers and initiator dissolved readily in the dichloromethane. The solution was vortexed for 1 min and sonicated for 30s at 25°C, after which no bubbles remained in the sample. The solution was allowed to sit for 30 min at 25°C and was subsequently injected between 2"x 3" glass microscope slides separated by 1" glass spacers and fastened together by binder clips. The glass slides were then placed in a UVP CL-1000L 365 nm UV crosslinking chamber and irradiated for 30 min, after which the cured thermoset gel was placed under vacuum at 80°C for 5h to remove the solvent and post-cured under vacuum at 150°C. The resulting thermoset polymer film was transparent and was of appropriate sample dimensions and sufficient mechanical integrity to be machined and subjected to numerous mechanical characterization experiments, including dynamic mechanical analysis and uniaxial tensile testing experiments.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective testing measurements.

Therefore, the present invention is well adapted to attain the ends and advantages mentioned as well as those that are inherent therein.

## Claims

1. A composition comprising a polycarbonate formed from polyhydroxyl monomers which are linked by a carbonate moiety,
wherein the polyhydroxyl monomers comprise a polyhydroxyl monomer of formula (1): 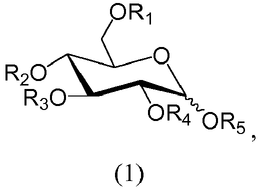
wherein R₁, R₂, R₃, and R₄ are each independently selected from H, a protecting group (e.g., a benzylic group, TBS, and TMS), or COCl;
R₅ is independently selected from H, a protecting group, COCl, or 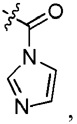 and wherein the polyhydroxyl monomer is not glucose.

2. The composition of claim 1, wherein the polycarbonate is according to the following formula: 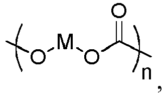 wherein O-M-O represents a polyhydroxyl monomer repeating unit and n is >1.

3. The composition of claim 1 or claim 2, wherein the polycarbonate is formed from more than one type of polyhydroxyl monomer.

4. The composition of any of claims 1-3 wherein the polycarbonate has a linear structure.

5. The composition of any of claims 1-3 wherein the polycarbonate has a hyperbranched structure.

6. The composition of any of claims 1-5 wherein the polyhydroxyl monomer of formula (1) is selected from:

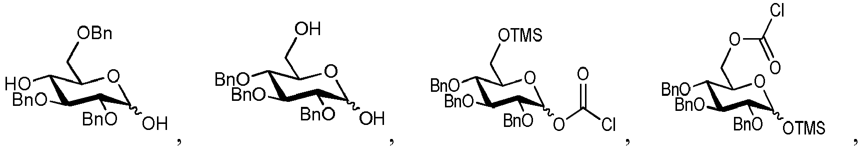

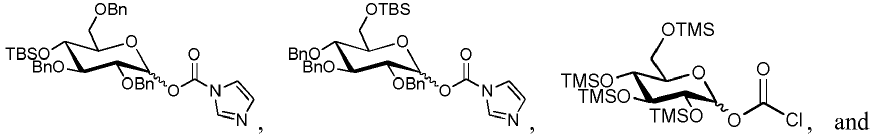

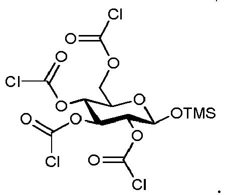

7. The composition of claim 1, comprising two or more polycarbonates as defined in any one of claims 1-6, wherein the two or more polycarbonates form a network.

8. The composition of claim 7, further comprising a crosslink between at least two linear polycarbonates.

9. A method comprising:
providing a polyhydroxyl monomer and a carbonylation agent; and
polymerizing the polyhydroxyl monomer in the presence of the carbonylation agent so as to form a polycarbonate,
wherein the polyhydroxyl monomer comprises a polyhydroxyl monomer of formula (1): 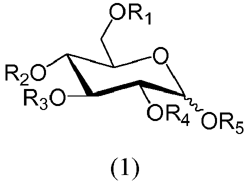 wherein
R₁, R₂, R₃, and R₄ are each independently selected from H, a protecting group (e.g., a benzylic group, TBS, and TMS), or COCl;
R₅ is independently selected from H, a protecting group, COCl, or 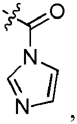 and wherein the polyhydroxyl monomer is not glucose.

10. The method of claim 9, wherein the polycarbonate is according to the following formula: 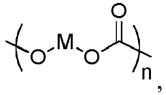 wherein O-M-O represents a polyhydroxyl monomer repeating unit and n is >1.

11. The method of any one of claims 9-10, wherein more than one type of polyhydroxyl monomer is present.

12. The method of any one of claims 9-11, wherein the carbonylation agent is selected from the group consisting of phosgene, diphosgene, triphosgene, derivaties of phosgene, carbonyldiimidazole, bis(4-nitrophenyl)carbonate, epoxide monomers, carbon dioxide, and combinations thereof.

13. The method of any one of claims 9-12, further comprising crosslinking the polycarbonate by forming one or more crosslinks between any of: two or more linear polycarbonates and two or more multifunctional comonomers.

14. The method of any one of claims 9-13, further comprising:
allowing the polycarbonate to undergo at least a partial hydrolysis.

## Patentansprüche

1. Zusammensetzung umfassend ein Polycarbonat, das aus Polyhydroxylmonomeren gebildet wird, die durch einen Carbonatanteil verknüpft sind,
wobei die Polyhydroxylmonomere ein Polyhydroxylmonomer der Formel (1) umfassen: 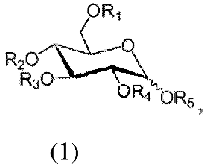
wobei R₁, R₂, R₃ und R₄ jeweils unabhängig ausgewählt werden aus H, einer Schutzgruppe (z.B. einer benzylischen Gruppe, TBS und TMS) oder COCl;
R₅ unabhängig ausgewählt wird ausH, einer Schutzgruppe, COCl oder 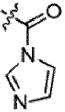 und wobei das Polyhydroxylmonomer nicht Glukose ist.

2. Zusammensetzung nach Anspruch 1, wobei das Polycarbonat der folgenden Formel entspricht: 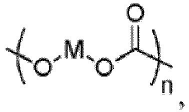 wobei O-M-O eine Polyhydroxylmonomer-Wiederholungseinheit darstellt und n > 1 beträgt.

3. Zusammensetzung nach Anspruch 1 oder Anspruch 2, wobei das Polycarbonat aus mehr als einem Typ Polyhydroxylmonomer gebildet wird.

4. Zusammensetzung nach einem der Ansprüche 1-3, wobei das Polycarbonat eine lineare Struktur aufweist.

5. Zusammensetzung nach einem der Ansprüche 1-3, wobei das Polycarbonat eine hyperverzweigte Struktur aufweist.

6. Zusammensetzung nach einem der Ansprüche 1-5, wobei das Polyhydroxylmonomer der Formel (1) ausgewählt wird aus: 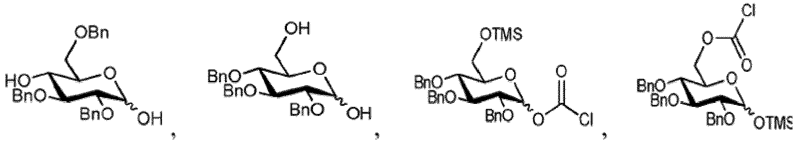 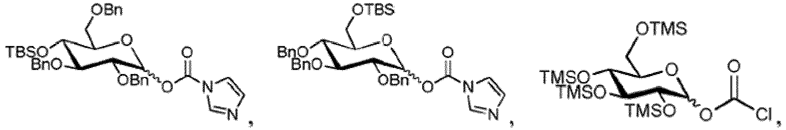 und

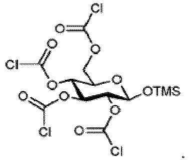

7. Zusammensetzung nach Anspruch 1, umfassend zwei oder mehr Polycarbonate wie in einem der Ansprüche 1-6 definiert, wobei die zwei oder mehr Polycarbonate ein Netzwerk bilden.

8. Zusammensetzung nach Anspruch 7, ferner eine Vernetzung zwischen mindestens zwei linearen Polycarbonaten umfassend.

9. Verfahren umfassend:
Bereitstellen eines Polyhydroxylmonomers und eines Carbonylierungsmittels; und
Polymerisieren des Polyhydroxylmonomers in Gegenwart des Carbonylierungsmittels, um ein Polycarbonat zu bilden,
wobei das Polyhydroxylmonomer einen Polyhydroxylmonomer der Formel (1) umfasst: 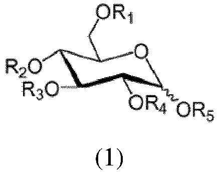 wobei
R₁, R₂, R₃ und R₄ jeweils unabhängig ausgewählt werden aus H, einer Schutzgruppe (z.B. einer benzylischen Gruppe, TBS und TMS) oder COCl;
R₅ unabhängig ausgewählt wird aus **H,** einer Schutzgruppe, COCl oder 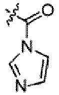 und wobei das Polyhydroxylmonomer nicht Glukose ist.

10. Verfahren nach Anspruch 9, wobei das Polycarbonat der folgenden Formel entspricht: 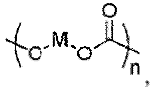 wobei O-M-O eine Polyhydroxylmonomer-Wiederholungseinheit darstellt und n > 1 beträgt.

11. Verfahren nach einem der Ansprüche 9-10, wobei mehr als ein Typ Polyhydroxylmonomer vorliegt.

12. Verfahren nach einem der Ansprüche 9-11, wobei das Carbonylierungsmittel aus der Gruppe ausgewählt ist bestehend aus Phosgen, Diphosgen, Triphosgen, Derivaten von Phosgen, Carbonyldiimidazol, Bis(4nitrophenyl)carbonat, Epoxidmonomeren, Kohlendioxid und Kombinationen davon.

13. Verfahren nach einem der Ansprüche 9-12, ferner das Vernetzen des Polycarbonats durch Bilden einer oder mehrerer Vernetzungen zwischen irgendwelchen von: zwei oder mehr linearen Polycarbonaten und zwei oder mehr multifunktionellen Comonomeren umfassend.

14. Verfahren nach einem der Ansprüche 9-13, ferner Folgendes umfassend:
Gestatten, dass das Polycarbonat mindestens eine partielle Hydrolyse durchmacht.

## Revendications

1. Composition comprenant un polycarbonate formé de monomères de polyhydroxyle qui sont liés par une fraction carbonate,
les monomères de polyhydroxyle comprenant un monomère de polyhydroxyle de formule (1) : 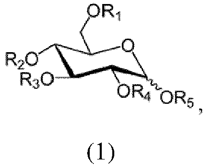
R₁, R₂, R₃, et R₄ étant chacun indépendamment sélectionnés parmi **H,** un groupe protecteur (par exemple, un groupe benzylique, TBS, et TMS), ou COCl;
R₅ étant indépendamment sélectionné parmi H, un groupe protecteur, COCl, ou 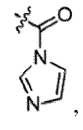 et le monomère de polyhydroxyle n'étant pas le glucose.

2. Composition selon la revendication 1, le polycarbonate étant selon la formule suivante : 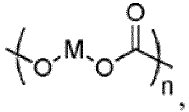 O-M-O représentant une unité de répétition de monomère de polyhydroxyle et n étant > 1.

3. Composition selon la revendication 1 ou la revendication 2, le polycarbonate étant formé de plus d'un type de monomère de polyhydroxyle.

4. Composition selon l'une quelconque des revendications 1 à 3, le polycarbonate ayant une structure linéaire.

5. Composition selon l'une quelconque des revendications 1 à 3, le polycarbonate ayant une structure hyper-ramifiée.

6. Composition selon l'une quelconque des revendications 1 à 5, le monomère de polyhydroxyle de formule (1) étant sélectionné parmi :

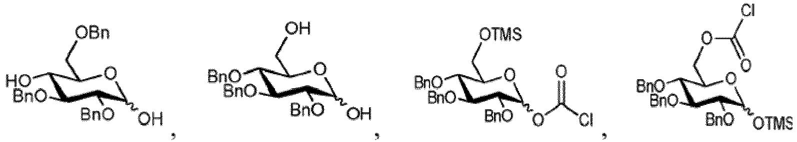

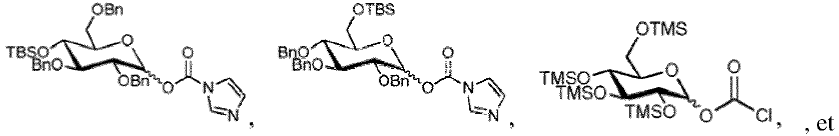

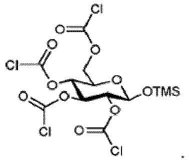

7. Composition selon la revendication 1, comprenant deux polycarbonates ou plus tels que définis selon l'une quelconque des revendications 1 à 6, les deux polycarbonates ou plus formant un réseau.

8. Composition selon la revendication 7, comprenant en outre une réticulation entre au moins deux polycarbonates linéaires.

9. Procédé comprenant :
la fourniture d'un monomère de polyhydroxyle et d'un agent de carbonylation; et
la polymérisation du monomère de polyhydroxyle en la présence de l'agent de carbonylation pour former un polycarbonate,
le monomère de polyhydroxyle comprenant un monomère de polyhydroxyle de formule (1) : 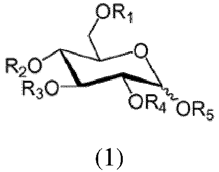
R₁, R₂, R₃, et R₄ étant chacun indépendamment sélectionnés parmi **H,** un groupe protecteur (par exemple, un groupe benzylique, TBS, et TMS), ou COCl;
R₅ étant indépendamment sélectionné parmi H, un groupe protecteur, COCl, ou 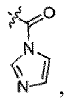 et le monomère de polyhydroxyle n'étant pas le glucose.

10. Procédé selon la revendication 9, le polycarbonate étant selon la formule suivante : 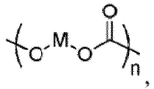 O-M-O représentant une unité de répétition de monomère de polyhydroxyle et n étant > 1.

11. Procédé selon l'une quelconque des revendications 9 à 10, plus d'un type de monomère de polyhydroxyle étant présent.

12. Procédé selon l'une quelconque des revendications 9 à 11, l'agent de carbonylation étant sélectionné parmi le groupe constitué du phosgène, du diphosgène, du triphosgène, des dérivés de phosgène, du carbonyldiimidazole, du bis(4-nitrophényl)carbonate, des monomères époxydes, du dioxyde de carbone, et de leurs combinaisons.

13. Procédé selon l'une quelconque des revendications 9 à 12, comprenant en outre la réticulation du polycarbonate par formation d'une ou plusieurs réticulations entre n'importe lequel parmi : deux polycarbonates linéaires ou plus et deux comonomères multifonctionnels ou plus.

14. Procédé selon l'une quelconque des revendications 9 à 13, comprenant en outre :
permettre au polycarbonate de subir au moins une hydrolyse partielle.
